## Europäisches Patentamt
## European Patent Office
## Office européen des brevets

(19)

(11) Veröffentlichungsnummer: **0 418 746 A1**

(12)

# EUROPÄISCHE PATENTANMELDUNG

(21) Anmeldenummer: 90117706.3

(22) Anmeldetag: 14.09.90

(51) Int. Cl.5: **A61B 3/16**

(30) Priorität: 22.09.89 DE 3931630

(43) Veröffentlichungstag der Anmeldung:
27.03.91 Patentblatt 91/13

(84) Benannte Vertragsstaaten:
AT BE CH DE DK ES FR GB GR IT LI LU NL SE

(71) Anmelder: DATRON-ELECTRONIC GmbH
In den Gänsäckern 10
W-6109 Mühltal-Traisa(DE)

(72) Erfinder: Hock, Bertram
Alois-Wenzel-Strasse 28
W-8751 Haibach(DE)

(74) Vertreter: Otte, Peter, Dipl.-Ing.
Tiroler Strasse 15
W-7250 Leonberg(DE)

(54) Verfahren und Vorrichtung zur Bestimmung des Augeninnendrucks o. dgl.

(57) Zur Bestimmung des Augeninnendrucks wird bei einem Tonometer, bei dem durch ein Meßprisma, welches mit vorgegebener Kraft auf das menschliche Auge drückt, ein entsprechender Zusammenhang zwischen der Kraft und der hierdurch applanierten Fläche hergestellt wird, vorgeschlagen, pro Zeiteinheit im Verlauf einer kontinuierlichen Druckerhöhung durch motorische, federvorgespannte Bewegung des Meßprismas eine Vielzahl von jeweils zueinander gehörenden Meßwerten von Kraft und applanierter Fläche zu ermitteln und in Form eines Kurvenverlaufs so aufzubereiten, daß sich aus der Vielzahl der Meßwerte differentiell der Augeninnendruck ermitteln läßt.

Fig.4

## VERFAHREN UND VORRICHTUNG ZUR BESTIMMUNG DES AUGENINNENDRUCKS O. DGL.

Stand der Technik

Die Erfindung geht aus von einem Verfahren nach dem Oberbegriff des Anspruchs 1 und einer Vorrichtung nach dem Oberbegriff des Anspruchs 15.

Allgemein betrifft die Erfindung das Gebiet der Feinmeßtechnik; sie ermöglicht bei ordnungsgemäßer Handhabung die lageunabhängige Kraftmessung speziell mittels eines sogenannten Tonometers, wobei die bei jedem Meßvorgang vorhandenen Erdanziehungskräfte nicht zur Auswirkung kommen oder jedenfalls durch entsprechende Vorkehrungen im Bereich der Hardware oder Software berücksichtigt werden können.

Um den Augeninnendruck bei Patienten messen zu können, wird einem allgemeinen Grundprinzip folgend üblicherweise so vorgegangen, daß mit einem geeigneten planen Teil gegen den als Kugelform zugrunde gelegten Augapfel gedrückt und bei Erreichen eines vorgegebenen Durchmessers der hierdurch applanierten Kreisfläche der Druck gemessen wird, der für das Zustandekommen dieses Durchmessers eingesetzt werden mußte.

Bei einem bekannten Gerät kommt dabei ein um einen stationären Drehpunkt verschwenkbarer Hebel zur Einwirkung, der unterhalb einer Stirnauflage (zur Erzielung einer festen Position) eine auf einem Kreisbogen basierende Schwenkbewegung in Richtung auf das Auge durchführt und dadurch mit meßbarer Krafteinwirkung gegen das Auge gedrückt werden kann. Am abgewandten Ende des Hebels auf der anderen Seite des Drehpunkts greift über eine vergleichsweise weiche Feder ein Elektromotor an und realisiert so die zur Durchführung der Messung erforderliche Annäherungsbewegung an das Auge.

Bei einer solchen bekannten Meßapparatur kann die Aufhängung des pendelartigen Hebels problematisch sein, da dieser aufgrund seiner Schwenkpunktlagerung einen Kreisbogen beschreibt. Es ist auch die Möglichkeit nicht auszuschließen, daß eventuelle Meßfehler infolge fehlender Auswuchtung im dynamischen Zustand auftreten. Ferner stoßen bei solchen Pendelapparaturen Miniaturisierungsbemühungen schnell an eine Grenze.

Es ist ferner schon ein Tonometer vorgeschlagen wor den (nicht vorveröffentlichte DE-OS 38 18 434 ), bei der zu beiden Seiten von Lagerrollen ein erster Meßschlitten und ein zweiter Gegenschlitten angeordnet sind, die miteinander eine mechanisch gekoppelte gegenläufige Bewegung ausführen, wobei die Andrückwirkung von Meßschlitten und Gegenschlitten auf die Lagerrollen durch eine aufeinander ausgeübte magnetische Wechselwirkung hervorgerufen wird. Die beiden Schlitten können daher eine freie gegenläufige Verschiebebewegung ausführen, ohne durch die jeweils einwirkende Gewichtskraft gehindert zu sein. Der Meßschlitten lagert dann einen Sensorkopf in Form eines Prismas, dessen Reflexionseigenschaften durch die Abplattung des Augapfels bei einwirkendem Druck eine Veränderung erfahren und von einem lichtempfindlichen Element erfaßt und bei Erreichen eines vorgegebenen Abplattungsdurchmessers zur Signalgabe ausgenutzt werden.

Insofern beruhen alle bisher bekannten Tonometersysteme auf dem Grundsatz der Durchführung einer Einzelmessung, wobei diese Einzelmessung vereinbarungsgemäß dann durchgeführt wird, wenn ein Applanationsdurchmesser von genau 3,06 mm erreicht ist; dieser Wert hat sich durch empirische Untersuchungen als besonders geeignet erwiesen, insbesondere deshalb, weil sich in einem bestimmten Bereich verschiedene Einflußfaktoren einigermaßen gegeneinander aufheben können, beispielsweise Adhäsionskraft und Membransteifigkeit (Aufsatz von Goldmann und Schmitt in DE-Ophthamalogica 134, 1957).

Ferner ist zu berücksichtigen, daß bei solchen Messungen eine möglichst gute Entkopplung der Meßgrößen Tränenflüssigkeitsmenge und applanierte Fläche erreicht wird, wobei auch schon vorgeschlagen wurde, eine sogenannte Nullapplanation durchzuführen, um eine Tränenflüssigkeitsmenge zu bestimmen, die dann über eine Ausgleichsrechnung in die Flächenermittlung mit einbezogen wird. Stets handelt es sich hierbei jedoch um die Notwendigkeit, Einzelmessungen (etwa für Nullapplanation und den schon erwähnten Durchmesser von 3,06 mm) durchzuführen, die allein durch zufällige Fehlereinwirkungen stark fehlerbehaftet sein können, wobei auch ein Fehlverhalten des Probanden im Moment der Messung das Meßergebnis stark verfälschen kann.

Der Erfindung liegt daher die Aufgabe zugrunde, eine Möglichkeit zur Bestimmung des Augeninnendrucks anzugeben, die in der Lage ist, ohne größere Belastung des Probanden stets ein überraschend einwandfreies, vor allen Dingen hochgenaues Meßergebnis zu liefern, ohne daß es erforderlich ist, die bei Einzelmessungen auftretenden, das Meßergebnis verfälschenden Imponderabilien verschiedener Genese einzubeziehen.

Vorteile der Erfindung

Die Erfindung löst diese Aufgabe mit den kennzeichnenden Merkmalen des Anspruchs 1 bzw. den kennzeichnenden Merkmalen des Anspruchs 15 und hat den Vorteil, daß es gelingt, durch die Realisierung eines vollständig unterschiedlichen Meßverfahrens den Einfluß zufälliger Fehler gering zu halten, und zwar dadurch, daß eine Vielzahl von Messungen pro Zeiteinheit durch geführt wird, wobei der eine Funktionswert, nämlich die auf den Augapfel einwirkende Kraft kontinuierlich geändert und durch eine geeignete Messung gleichzeitig mit dem sich entsprechend ändernden Applanationsdurchmesser des Auges in dieser korrelierten Form tabellenartig gespeichert wird, so daß es gelingt, eine Art Applanationsfunktion aufzunehmen.

Hierzu kann mit Vorteil auf ein vergleichsweise breites Band von Applanationsdurchmessern abgestellt werden, in welchen sich die verschiedenen Einflußfaktoren praktisch gegenseitig aufheben, so daß man in diesem Band den Augeninnendruck durch Messung richtig bestimmen kann, wenn die gemessene Kraft mit der gemessenen Fläche in Relation gesetzt wird. Zu diesem Zweck wird der bevorzugte Bereich von Applanationsdurchmessern langsam bei der Vielfachmessung nach vorliegender Erfindung durchfahren und in einer Tabelle werden alle stichprobenartig pro Zeiteinheit ermittelten Kraftwerte in Abhängigkeit der ebenfalls ermittelten Fläche gespeichert, woraufhin es möglich ist, nach dieser eigentlichen, insofern einen Kennlinienverlauf mit sehr vielen Meßwerten darstellenden Kurve die Messung durch die Bildung eines Differenzenkoeffizienten auszuwerten, also aus den aufgenommenen Meßwerten den Augeninnendruck differentiell zu bestimmen. In diesem Zusammenhang ist es auch möglich, durch die aufgezeichneten, beispielsweise in einem Speicher geeigneten Umfangs eines Mikrorechners niedergelegten Tabellenwerten eine Ausgleichsgerade zu legen, in deren Steigung der zu ermittelnde Innendruck implizit enthalten ist.

Durch ein solches Vorgehen gelingt es ferner, einen als eventuellen Lagefehler sich manifestierenden Kraftoffset sowie den auf die Tränenflüssigkeit zurückzuführenden Flächenoffset zu definieren und durch entsprechende Rechnung zu eliminieren.

Durch die in den Unteransprüchen aufgeführten Maßnahmen sind vorteilhafte Weiterbildungen und Verbesserungen der Erfindung möglich. Besonders vorteilhaft ist die absolut reibungsfreie Aufhängung des den Meßkörper bildenden Prismas, wobei die von dem Prisma auf das Auge ausgeübte Kraft über die Auslenkung einer Feder bestimmt wird, die ihrerseits wieder absolut rückwirkungsfrei optisch gemessen wird.

Eine durch die Lagerfeder gleichzeitig realisierte Parallelogrammführung letztlich des Meßkörpers (Prisma) sichert eine auf den Meßweg bezogene gute Geradführung des Meßkörpers und verhindert bzw. optimiert den Einfluß eventueller minimaler seitlicher Bewegungen bei der Auslenkung des Meßkörpers durch ihre eigene Auslegung.

Die vollständig reibungsfreie Aufhängung des Meßkörpers kann auch während des Meßvorgangs zu geringfügigen Schwingungsbewegungen führen, wobei durch die insgesamt elektronische Auswertung des Meßkurvenverlaufs (Abhängigkeit der Applanationsfläche bzw. deren Durchmessers von der zur Einwirkung gebrachten Kraft) solche statistischen Schwingungen um den eigentlichen Kurvenverlauf werden durch die durch die Erfindung realisierte Vielfachmessung problemlos im Software bereich berücksichtigt und eliminiert werden; ergänzend hierzu ist es aber noch möglich, die Schwingung des Sensors mit der Meßfeder durch eine geeignete mechanische Dämpfung, beispielsweise Luftkammer oder Dämpfung durch Wirbelstrombremse gering zu halten.

Durch die hier insgesamt neue Grundkonzeption eines Tonometers ergeben sich noch eine Vielzahl weiterer Vorteile, die aus Gründen der Übersichtlichkeit dann jeweils sinnvollerweise in Verbindung mit der nachfolgenden Beschreibung erläutert werden.

Zeichnung

Ein bevorzugtes Ausführungsbeispiel vorliegender Erfindung ist in der Zeichnung dargestellt und wird in der nachfolgenden Beschreibung näher erläutert. Es zeigen:

Fig. 1 die spezielle, in einem Gehäuse untergebrachte Meßapparatur einer Ausführungsform des erfindungsgemäßen Tonometers in einem ersten Längsschnitt längs der Linie I-I der Fig. 3;

Fig. 2 ebenfalls einen Längsschnitt der Ausführungsform der Fig. 1 um 90° gedreht längs der Linie II-II der Fig. 1;

Fig. 3 einen Querschnitt durch die Ausführungsform der Figuren 1 und 2 längs der Linie III-III der Fig. 1;

Fig. 4 in Form eines Diagramms als Kennlinie die Abhängigkeit des Durchmessers der applanierten Fläche in Abhängigkeit zur einwirkenden Kraft;

die Figuren 5-8 mögliche Ausführungsformen von den Meßkörper bildenden Prismen;

EP 0 418 746 A1

Fig. 9 in vergrößertem Maßstab den Teilausschnitt X der Fig. 1;

Fig.10 schematisiert den Prismen-Meßkörper mit zugeordneten Linsen, Strahlungsquelle und Strahlungsempfänger als Ausschnitt;

Fig.11 eine mögliche Ausführungsform eines Prismas als Meßkörper mit Teilen der Lagerung in perspektivischer Darstellung und

Fig.12 das gleiche Prisma in einer Ansicht von vorn mit Darstellung geschwärzter Teilbereiche zur Erleichterung der Positionierung vor dem Auge des Probanden;

die Figuren 13 und 14 in Seitenansicht und Frontansicht stark schematisiert eine universelle, von einem Rechner oder durch manuelle Bedienung steuerbare Kopfabstützung am Gehäuse des erfindungsgemäßen Tonometers;

Fig.15 eine weitere Ausführungsform einer rechnergesteuerten Kopfabstützung und

Fig.16 eine mögliche Ausführungsform der Organisation des Mikrorechners für die Softwareauswertung der gewonnenen Meßwerte.

Beschreibung der Ausführungsbeispiele

Der Grundgedanke vorliegender Erfindung beruht auf der Erkenntnis, daß es nur durch eine kontinuierliche Meßwertgewinnung, die der Darstellung einer Vielzahl korrelierter Tabellenwerte in Kurvenform entspricht, überhaupt möglich ist, die dem Fachmann hinreichend bekannten, zu zum Teil erheblichen Meßfehlern führenden verschiedenen Einflußfaktoren so zu beherrschen, daß man aus der durch zufällige Fehler stark beeinflußten Einzelmessung herauskommt und immer ein hochgenaues Endergebnis erzielen kann.

Daher sieht die erfindungsgemäße Meßapparatur die Möglichkeit vor, die auf den Augapfel einwirkende Kraft kontinuierlich zu erhöhen, so daß in Verbindung mit der ständigen Messung des jeweils momentanen Applanationswerts letztlich eine Applanationsfunktion gewonnen werden kann, aus welcher der Augeninnendruck dann differentiell auf der Basis der Vielzahl der aufgenommenen Meßwerte bestimmt werden kann. Hierdurch ergibt sich schon der erhebliche Vorteil bezüglich der Einflußwirkung zufälliger Fehler, die, wie ja für sich gesehen bekannt, mit der Wurzel aus der Meßpunktanzahl abnehmen.

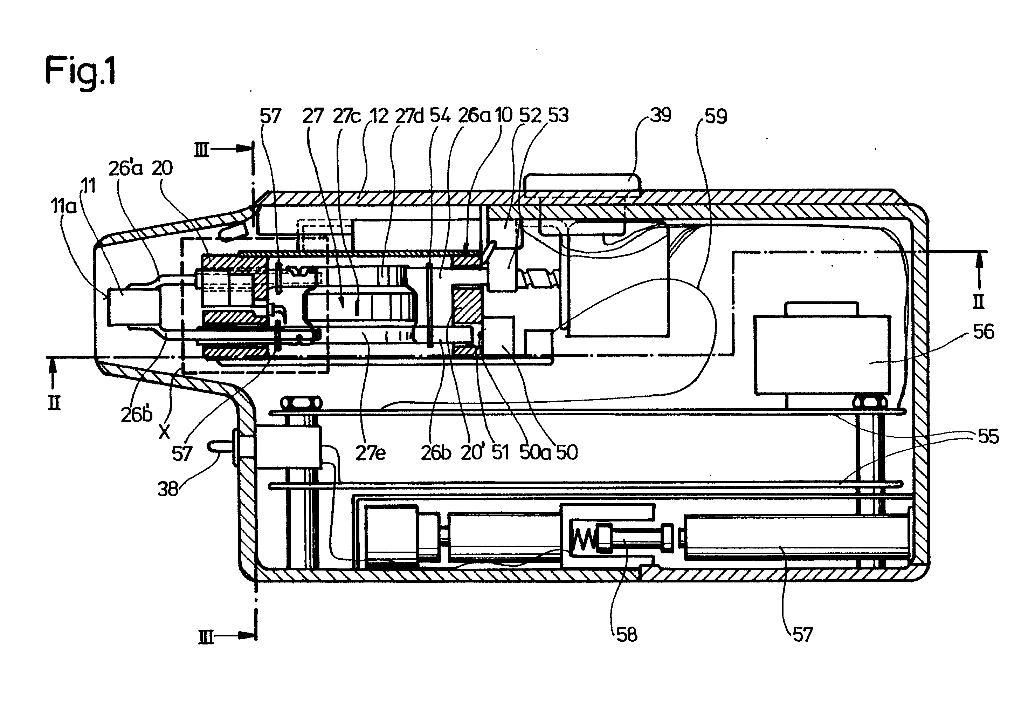

Bei der Erfindung ist daher, wie die Figuren 1, 2 und 3 zeigen, ein motorisch bewegbarer Schlitten 10 vorgesehen, der seinerseits wieder den relativ zu ihm beweglich unter Federkraftwirkung verschiebbaren Meßkörper lagert, der in den Figuren als Prisma 11 angegeben ist. Hierdurch ist es möglich, bei stationär am Auge anliegendem Prisma 11 die Druckkraft durch die Schlittenverschiebung kontinuierlich zu erhöhen, gleichzeitig eine optische Messung des jeweils erreichten Applanationsdurchmessers durchzuführen und aus der Reaktion der federnden Lagerung des Prismas unter Einbeziehung der Federkennlinie die dabei an wachsende Kraft, vorzugsweise ebenfalls durch ein optisches Meßverfahren, zu erfassen. Der Aufbau eines bevorzugten Ausführungsbeispiels einer solchen Meßapparatur (Tonometer) für die Durchführung einer multiplen Meßwertgewinnung ist dabei im einzelnen wie folgt getroffen.

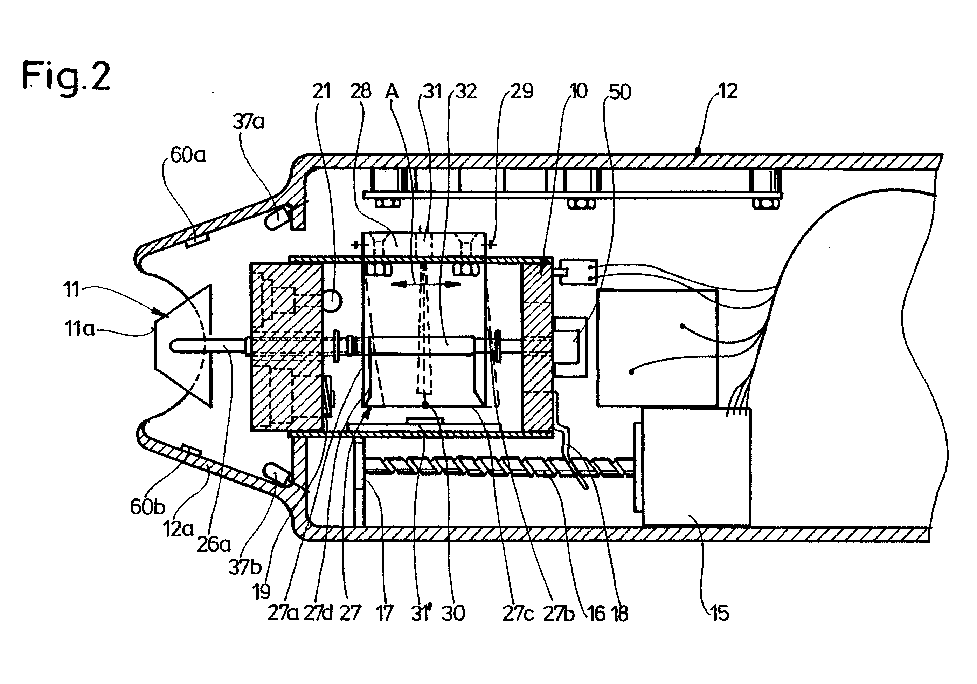

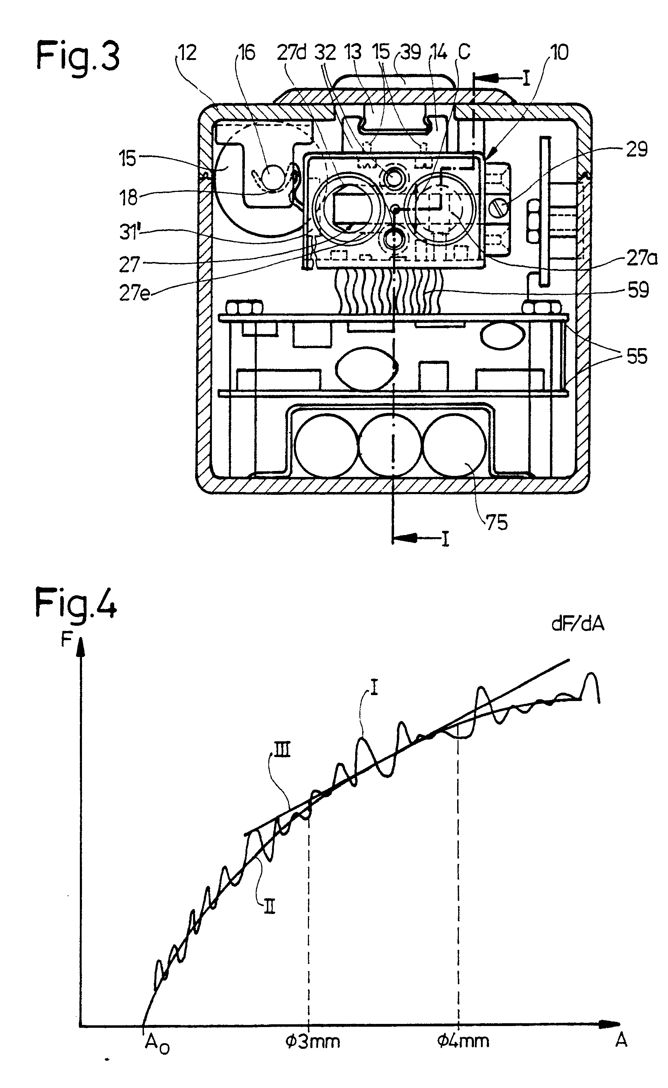

Der Schlitten 10 ist in geeigneter Weise am umgebenden Gehäuse 12 des Tonometers verschieblich gelagert, und zwar bei dem dargestellten Ausführungsbeispiel, wie am besten der Darstellung der Fig. 3 entnommen werden kann, mittels einer am Gehäuse ortsfesten Führungsschiene 13, die von einem am Schlitten 10 in geeigneter Weise, beispielsweise mittels Schrauben 15 gehaltenen Gleitkörper 14 umfaßt wird. Die jeweils komplementäre Form von Führungsschiene 14 und Gleitkörper 15 kann dabei schwalbenschwanzähnlich ausgebildet sein, wobei hier natürlich auch andere Linearführungen in Frage kommen, auf die nicht weiter eingegangen zu werden braucht. Der Schlitten 10 ist daher in der Lage, wie in Fig. 2 gezeigt, in Richtung des Doppelpfeils A eine Relativverschiebung im Gehäuse durchzuführen, und zwar unter der Wirkung eines motorischen Antriebs, wozu bei dem dargestellten Ausführungsbeispiel ein Elektromotor 15 vorgesehen ist, der über eine von ihm angetriebene Ausgangsspindel 16 mit Gegenlager bei 17 den Schlitten 10 dadurch antreibt, daß in das Gewinde der Spindel 16 ein am Schlitten 10 ortsfestes Führungsglied 18, vorzugsweise in Form einer Feder eingreift. Es versteht sich, daß der Antrieb bzw. der Vorschub des Schlittens 10 auch durch andere Transportmittel erfolgen kann, bei spielsweise über einen vorzugsweise durch Ausgleichsfedern vorgespannten Seilzug, wodurch auch noch Körperschallübertragungen vermieden werden können.

Der Schlitten 10 trägt dann ferner noch die Flächenmeßeinrichtung (für die jeweils realisierte Applanationsfläche des Auges), die Kraftmeßeinrichtung, durch welche die Kraft bestimmt werden kann, mit der das Prisma 11 auf den Augapfel drückt, sowie eine mögliche Ausführungsform einer Positioniereinrichtung, um das Prisma relativ zum Auge ordnungsgemäß positionieren zu können.

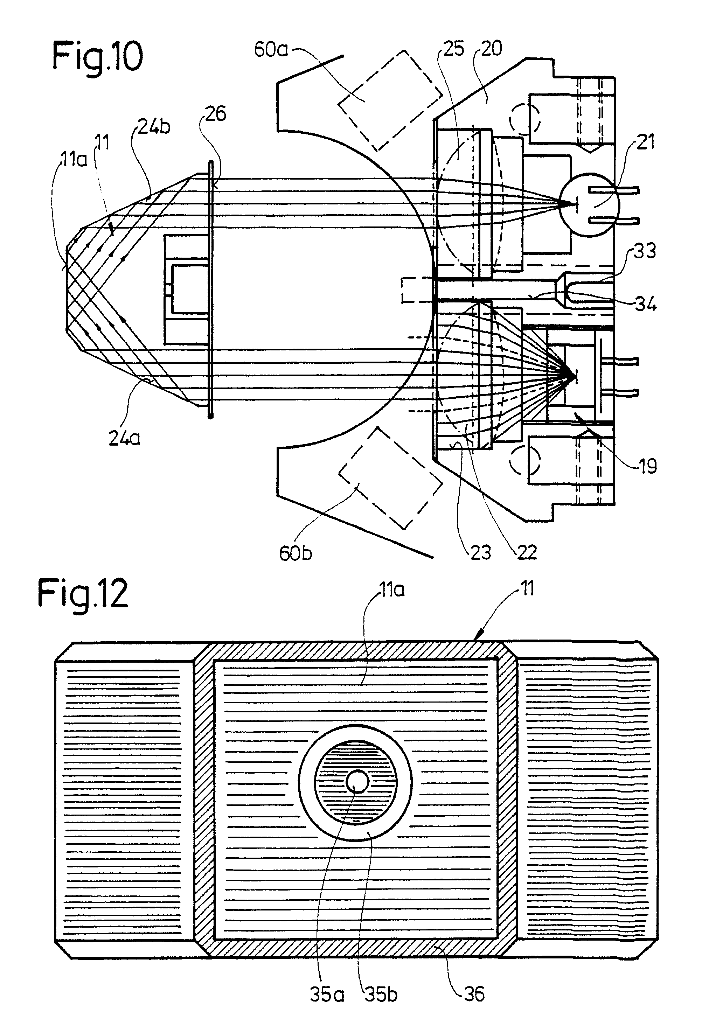

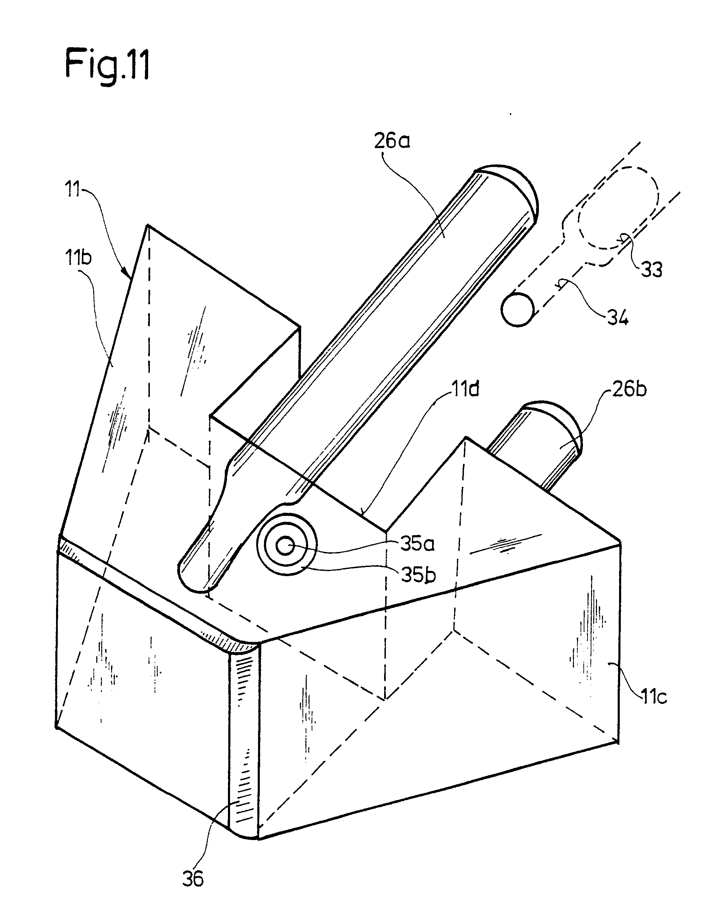

Die Flächenmeßeinrichtung ist schematisiert in Fig.10 besonders übersichtlich dargestellt und umfaßt

4

neben dem schon erwähnten Prisma 11 einen Lichtsender in Form einer Infrarotlicht-Leuchtdiode (IR-LED) für das Prisma, die das Bezugszeichen 19 trägt und die in einer geeigneten Ausnehmung eines Optik-Lagerblocks 20 gehalten ist, zusammen mit dem Licht- oder Fotoempfänger 21 etwa in Form eines geeigneten Fototransistors.

Um hier überhaupt zu verwertbaren Meßergebnissen zu kommen, ist folgendes zu berücksichtigen. Körper, deren Brechungsindices mindestens gleich dem des Wassers sind, heben die Totalreflexion bei der Berührung der vorderen Prisma-Meßfläche 11a auf, so daß es durch eine Lichtmengenmessung möglich ist, zu einer Flächenbestimmung des applanierten Auges zu gelangen. Hierbei ist es aber erforderlich, daß von der IR-LED 19 paralleles, homogenes Licht erzeugt und ausgesandt wird, wozu dem Lichtsender eine asphärische Linse 22 zur Parallelbündelung der Lichtstrahlen nachgeschaltet ist, die in einer geeigneten Lagerausnehmung 23 des Optikblocks 20 aufgenommen ist. Von dieser Linse 22 gelangt das Licht durch Mehrfachbrechung an den schrägen Seitenflächen 24a, 24b und dem durch den jeweils applanierten Augenflächenbereich beeinflußten Reflexionsverhalten der Vorderfläche 11a des Prismas zurück zum Lichtempfänger 21, wobei eine weitere asphärische Linse 25 passiert wird, die das parallele Lichtbündel dann wieder auf den fotoempfindlichen Bereich des Fototransistors konzentriert. Die Linse 25 wirkt daher als Kondensorlinse für den nachgeschalteten Fotoempfänger, der natürlich auch eine geeignete Fotodiode sein kann.

Alternativ ist es auch möglich, ein entsprechend großflächiges Fotoelement falls gewünscht unmittelbar auf das Prisma aufzubringen, beispielsweise im Bereich der Rückfläche 26 (s. Fig. 10), wobei dann entsprechende Anschlußdrähte vorzugsweise längs der Prismahalterung zu führen sind. Tatsächlich ist es bei dem dargestellten Ausführungsbeispiel entsprechend den Figuren 1, 2, 3 und 10 so, daß das Meßprisma 11 sich relativ zu dem Optikblock 20 bewegt, der stationär fest am Schlitten 10 befestigt und von diesem getragen ist (s. Fig. 1). Durch die parallele Strahlungsführung mit Hilfe der asphärischen Linsen 22, 25 ergeben sich hierdurch keine Meßfehler.

Dabei kann im Hinblick auf die Anordnung eines Fotoelements direkt auf dem Prisma sofort darauf hingewiesen werden, daß die in diesem Fall verwendeten sehr dünnen Anschlußdrähte, die über die Prismenhalterung und die diese lagernde Meßfeder geführt werden, auch im Bereich der Meßfeder höchstens zu einer Veränderung der Federkennlinie führen, die jedoch als konstanter Faktor dann im Softwarebereich problemlos berücksichtigt werden kann.

Die grundsätzliche Funktion der solchermaßen ausgebildeten Flächenmeßeinrichtung (für die Augapfelapplanation) ist an sich schon in der weiter vorn erwähnten DE-OS 38 18 434 erläutert und beruht darauf, daß an der Frontfläche des Meßprismas so lange Totalreflexion herrscht, wie dessen vordere Fläche 11a das Auge noch nicht berührt. Sobald dann die Berührung erfolgt ist - hier ist dann auch noch der Einfluß der Tränenflüssigkeit zu berücksichtigen, worauf weiter unten eingegangen wird -, verringert sich die reflektierte Lichtmenge zunehmend mit zunehmender Auflage des Prismas auf das Auge und entsprechend zunehmendem Abplattungsbereich, so daß man nach vorhergehender Eichung den jeweils erreichten Applanationsdurchmesser bestimmen kann. Daher ist die Fläche des menschlichen Auges, die an dem Prisma applaniert wird, auf die vom Lichtempfänger 21 aufgenommene Lichtmenge bezogen, denn im Falle einer Applanation verringert sich wie erwähnt die Reflexion und streut im flachen Winkel vom Prisma weg. Diese von dem IR-Empfänger gemessene Verringerung ergibt ein Maß für die applanierte Fläche.

Um kontinuierlich fortlaufend die jeweils korrelierten Werte von Kraft und Applanationsdurchmesser bestimmen zu können, ist das Prisma 11 im Schlitten 10 federnd aufgehängt und wird also bei zunehmendem Vorschub des Schlittens 10 über den motorischen Antrieb mit seiner Lagerung in der gleichen Weise zunehmend gegen die Kraft seiner Aufhängefeder zurückgedrückt, wodurch diese Kraft zunimmt.

Zur Lagerung des Prismas 11 am Schlitten ist dieses daher, wie am besten die Darstellung der Fig. 1 zeigt, mit seiner oberen und seiner unteren Fläche an einer Prismenhalterung befestigt, die bei dem dargestellten Ausführungsbeispiel aus einem oberen Lagerrohr 26a und einem unteren Lagerrohr 26b besteht. Diese beiden Lagerrohre 26a, 26b durchsetzen sowohl den Optikblock 20 als auch entsprechend einem bevorzugten Ausführungsbeispiel eine hintere Abschlußwand 20′ des Schlittens 10 und sind auf dem Schlitten lediglich durch eine spezielle Meßfeder, sonst jedoch frei verschieblich und insofern auch absolut reibungsfrei aufgehängt. Diese Meßfeder, auf deren Aufbau gleich noch eingegangen wird, wirkt dann in Verbindung mit einem Positionssensor, wodurch die auf das menschliche Auge aufgebrachte Druckwirkung gemessen werden kann. Es ist also der Schlitten, der zunächst (prozessorgesteuert) von einem Antriebs-Schrittmotor gesteuert das Prisma in die Meßposition bringt und anschließend beim Weiterfahren die Relativverschiebung zwischen ihm und dem Meßprisma über die federnde Aufhängung hervorruft, wodurch die Kraftmessung möglich ist, die gleichzeitig abläuft mit der soeben schon geschilderten Flächenmessung durch Applanation des menschlichen Auges. Bei dieser ist das Prisma dann praktisch zum Stillstand gekommen und die auf das Auge einwirkende, insofern ansteigende Kraft wird von der Meßfeder erzeugt, umgesetzt aus der weiter ablaufenden Bewegung des Schlittens.

Im einfachsten Fall kann diese Kraftmessung so ablaufen, daß die zusätzliche aufzubringende Kraftkomponente bestimmt wird, die bei der weiteren Bewegung des Schlittens anfällt, wenn das Prisma zum Stillstand gekommen ist; da hier allerdings eine Vielzahl nicht mehr akzeptabler sonstiger Einwirkungen (Reibung u. dgl.) mit zur Auswirkung kommen würden, wird entsprechend einem bevorzugten Merkmal vorliegender Erfindung zur ausschließlichen Bestimmung der reinen, vom Meßprisma ausgehenden Kraft vorgeschlagen, diese über die erfolgte Auslenkung der Meßfeder zu bestimmen unter Einbeziehung der bekannten Federkonstanten.

Obwohl die hierbei verwendete Feder einen grundsätzlich beliebigen Aufbau haben könnte, wird vorgezogen, die Feder nach Art einer Parallelogrammführung auszubilden, so daß die Meßfeder 27, wie am besten der Darstellung der Fig. 2 entnommen werden kann, über zwei Hauptschenkel 27a, 27b verfügt, die am Schlitten mittels eines Lagerblocks 28 befestigt sind, beispielsweise durch seitliche Verschraubungen 29, wie am besten der Fig. 3 entnommen werden kann. Diese beiden Hauptschenkel 27a, 27b erstrecken sich quer zur Bewegungsrichtung A des Schlittens 10 und vereinigen sich einstückig an ihrem zur Einspannung abgewandten Ende über einen Basissteg 27c. Dieser Basissteg trägt gleichzeitig eine Art Schlitzblende 30 oder eine sonstige geeignete Öffnung, durch welche zur Bildung einer Positioniereinrichtung Licht von einer ebenfalls im Lagerblock 28 angeordneten Beleuchtungseinrichtung, fällt - hierauf wird weiter unten noch eingegangen.

Ferner gehen von dem Basissteg 27c der einstückigen Meßfeder 27 in der Zeichenebene nach oben und unten, also beidseitig zusätzliche einstückige Lagerlappen 27d, 27e ab, die mit einer Breite, die etwa der Länge des Basisstegs 27c entspricht, in Richtung der Hauptschenkel 27a, 27b um eine vorgegebene Länge zurückgeklappt sind bis zu den an dieser Stelle durchgeführten oberen und unteren Lagerrohren 26a, 26b für das Meßprisma 11 und mit diesen an dieser Stelle verbunden sind, beispielsweise indem sie diese, wie in Fig. 3 gezeigt, bei 32 umschlingen.

Hierdurch ergibt sich eine präzise Geradführung für die Prismenhalterung (aus den Rohren 26a, 26b) aufgrund der Parallelogrammführung der beiden Meßfeder-Hauptschenkel 27a, 27b und gleichzeitig über den Basissteg 27c die Kraftmeßeinrichtung für die Federverschiebung. Diese beruht auf dem Grundprinzip eines Positionssensors, realisiert durch die IR-Sendediode 31 und einen Lichtempfänger 31$'$ auf der gegenüberliegenden Seite der Schlitteninnenwandung, zwischen denen der Schlitz 30 des Meßfeder-Basisstegs 27c angeordnet ist. Eine solche eine Verschiebebewegung erfassende lichtoptische Anordnung - und mit Verschiebebewegung ist hier die Auslenkung der Meßfeder 27 be zeichnet - ist für sich. gesehen bekannt, wobei der Lichtempfänger 31$'$ ein flächiges Foto-Widerstandselement sein kann, welches je nachdem, wo durch die Schlitzblende 30 der Meßfeder 27$'$ das Licht der Sende-LED 31 auffällt, einen entsprechend unterschiedlichen Strom abgibt; solche Lichtempfänger werden im Fachgebrauch üblicherweise als sogenannte PSD-Elemente bezeichnet und werden im folgenden auch weiter so benannt. Daher ist es möglich, die in Fig. 2 strichpunktiert angedeutete mögliche Auslenkung des Meßfeder-Parallelogramms durch die Positionsveränderungen der Schlitzblende 30 über dem PSD-Element 31$'$ zu erfassen und in der zugeordneten Auswerteeinrichtung (Mikrorechner) in Kraftwerte umzurechnen, die dann tabellarisch den beispielsweise als Adressen gehandhabten Flächenwerten der Applanation zugeordnet werden, zur Realisierung der Applanationsfunktion in Form eines Kurvenverlaufs.

Schließlich gehört in diesen Bereich, da ebenfalls auf dem Schlitten montiert, noch eine erste Positioniereinrichtung, die dazu dient, die gesamte Meßapparatur mit Meßkörper, also dem Prisma 11 sehr genau so vor und auf das Auge ausgerichtet zu positionieren, daß die Messung in der gewünschten Weise und mit entsprechend hoher Genauigkeit durchgeführt werden kann. Bei dieser ersten Positioniereinrichtung erfolgt die Positionierung des Geräts vor dem Auge mit Hilfe einer geeigneten Lichtquelle 33, beispielsweise Positionier-LED, die, wie Fig. 10 am besten zeigt, ebenfalls zusätzlich noch im Optikblock 20 gelagert ist, und zwar auf der Achse der Flächennormale des Meßpris mas 11. Von dieser Positionier-LED fällt dann (gepulstes) Licht durch das Meßprisma 11 auf die Cornea, also die Augenoberfläche, wobei dieses Licht zunächst eine spezielle Struktur auf dem Meßkörper (Prisma 11) durchsetzt, bevor es ins Auge fällt. Diese spezielle Struktur läßt sich am besten der Darstellung der Figuren 11 und 12 entnehmen und besteht darin, daß bei in diesem Fall dann vorausgesetzter Schwärzung der beiden Prismaseitenflächen 11b und 11c sowie der dem Auge abgewandten, bei dem Ausführungsbeispiel der Fig.1 von einer Einbuchtung gebildeten Prismarückfläche 11d in dieser Prismarückfläche die Struktur von beispielsweise zwei konzentrischen Kreisen 35a als Innenkreis und 35b als Außenkreis gebildet ist. Durch die Schwärzung der Prismaseitenflächen 11b und 11c sowie der Prismarückfläche 11d, deren Breite und Höhe in etwa den Abmessungen der Frontfläche 11a des Prismas entspricht, ergibt sich für den Betrachter eine insofern vollständig geschwärzte Struktur des Meßprismas entsprechend Fig. 12, wobei alle sichtbaren Flächen, wie die vorgenommene Spezialschraffur andeutet, schwarz bleiben bis auf die beiden konzentrischen Kreise 35a, 35b, die von der Positionier-LED angestrahlt sind. Auf diese Weise läßt sich aufgrund der erkannten Helligkeitsunterschiede und der Form der gesehenen Figur (konzentrische Kreise) eine Positionierung des Gerätes auch bei eingeschränktem Gesichtsfeld vornehmen, da es lediglich notwendig ist, das Gerät so an das Auge heranzuführen, dessen Innendruck zu bestimmen ist, daß man mit diesem Auge die konzentrischen Kreise 35a, 35b stets voll beleuchtet erkennen kann. Der Grund für die Möglichkeit, eine Feinpositionierung auf diese Weise vorzunehmen, liegt darin, daß durch die konzentrischen Kreise 35a, 35b die Lichtquelle 33 gesehen werden kann, jedoch nur dann, wenn die Lichtquelle, der Kreismittelpunkt und die optische Achse der Blickrichtung auf einer Geraden liegen. Daher nehmen bei einer nichtkorrekten Positionierung sowohl die Intensität des Lichtes als auch die korrekte Gestalt der Kreise ab, so wie sie vom Auge gesehen werden. Hierdurch wird eine Feinjustage ermöglicht, die in Verbindung mit einer weiteren Positionierhilfe einen Einsatz des Geräts auch bei solchen Probanden möglich macht, bei denen ein teilweiser Gesichtsfeldausfall vorliegt oder stark geweitete, beispielsweise medikamentös bedingte, Pupillen vorliegen. Eine zweite Positionierhilfe besteht darin, daß die Umrandung der vorderen Fläche 11a des Meßprismas 11 mattiert ist und einen gebrochenen Kantenumlauf 36 aufweisen kann (Figuren 11 und 12).

Ferner sind gesonderte weitere Beleuchtungsquellen vorgesehen, die in Fig. 2 mit 37a, 37b bezeichnet sind und die, falls gewünscht, auch strahlen, etwa grünes Licht aussenden können. Daher ermöglicht dieser mattierte, etwa von der grünen Farbe beleuchtete Rand zunächst eine Grobjustage, wobei dann die in der Achse der Flächennormale des Meßprismas 11 liegende zentrale Lichtquelle 33 in Verbindung mit der Schwärzung und der speziellen optischen Struktur die Feinpositionierung ermöglicht sowohl hinsichtlich der Ausrichtung der Meßapparatur zum Auge als auch mit Bezug auf den Neigungswinkel. Dabei werden die konzentrischen Kreise 35a, 35b nicht vom Licht der zusätzlichen Beleuchtung 37a, 37b beleuchtet, wobei die Unterscheidung auch durch die verschiedene Farbgebung der Lichtquellen möglich ist.

Die zusätzliche Beleuchtung hat noch einen weiteren Sinn, der darin liegt, daß hierdurch eine Verkleinerung der Pupille durch das zusätzliche Licht erfolgt, welches ins Auge fällt, so daß die Positionierung mit Hilfe der konzentrischen Kreise noch genauer wird.

Der grundsätzliche Meßablauf ist dann wie folgt. Zunächst wird durch einen an der Vorderseite des Geräts noch vorgesehenen Hauptschalter 38 das Tonometer eingeschaltet und anschließend der Meßvorgang durch kurzes Drücken einer sich an der Oberseite des Geräts befindenden Start/Stop-Tasters 39 gestartet. Es beginnen dann die Positionier-Leuchtdiode 33 und die beiden zusätzlichen Leuchtdioden 37a, 37b zu leuchten; das Tonometer wird an das Auge herangeführt und positioniert.

Anschließend wird der Start/Stop-Taster 39 ständig gedrückt; die Positionier-LED 33 blinkt kurz auf und der Schlitten 10 fährt vor; es erfolgt dann eine Messung von maximal 5 Sekunden - auf den genauen Meßablauf wird weiter unten noch gesondert eingegangen; anschließend blinkt die Posionier-Leuchtdiode 33 erneut kurz auf, der Schlitten fährt zurück, und das Tonometer wird vom Auge weggeführt. Man kann dann den Meßwert an einer digitalen Anzeige an geeigneter Stelle der Gehäusewandung sofort ablesen und dann eventuell, falls gewünscht, einen weiteren Meßvorgang starten oder auch das Tonometer wieder ausschalten.

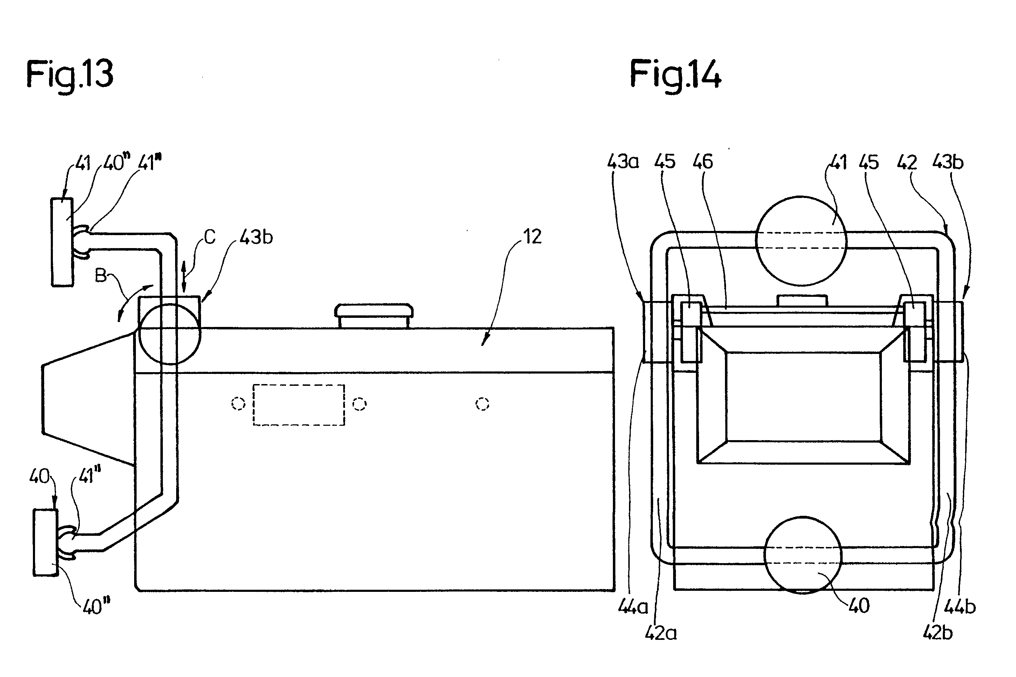

Ferner sind noch Positioniereinrichtungen im weiteren Sinne vorgesehen, von denen eine erste Ausführungsform in den Figuren 13 und 14 dargestellt ist.

Da es schwierig ist, das Gerät lediglich durch Festhalten mit der Hand vor dem Auge zu positionieren, sind an dem Gerät Kopfabstützungen vorgesehen, die entsprechend den Figuren 13 und 14 jeweils beispielsweise eine Wangenstütze 40 und eine Stirnstütze 41 umfassen.

Es ist zweckmäßig, die beiden Stützen 40, 41, die auch eine geeignete Polsterung umfassen können, mittels eines gemeinsamen, geschlossenen Bügels 42 zu lagern, der, wie Fig. 14 zeigt, eine rechteckförmige Grundform aufweisen kann und beidseits über Lagermittel 43a, 43b am Gehäuse 12 des Tonometers befestigt sein kann. Die Befestigungsmittel 43a, 43b können so ausgebildet sein, daß die seitlichen Bügelschenkel 42a, 42b durch Hülsen 44a, 44b geführt sind, die ihrerseits wieder entsprechend dem Pfeil B am Gehäuse 12 verschwenkbar befestigt sind, wobei die beiden Schenkel 42a, 42b des Lagerbügels 42 für die Wangen und die Stirnstütze in den Hülsen 44a, 44b auch in einer Richtung von oben nach unten entsprechend dem Doppelpfeil C verschiebbar sein können. Den Führungshülsen 44a, 44b können dann noch Feststelleinrichtungen 45 zugeordnet sein, die beispielsweise Schraubmittel umfassen, so daß sowohl die Bügelschenkel in den Hülsen festgeklemmt als auch die Hülsen in ihrer jeweiligen Winkelposition relativ zum Gehäuse 12 festgelegt werden können. Hierdurch erzielt man eine sichere Positionierung und Fixierung des Gehäuses 12 des Tonometers vor dem Auge, wobei es sich versteht, daß die Feststelleinrichtung 45 durch eine gemeinsame, manuell zu betätigende Klemmstange 46 betätigt werden kann oder auch von dem vorhandenen Mikrorechner durch entsprechende Ansteuerung das Festklemmen von Stirn- und Wangenstütze in der gewünschten Position dann vorgenommen werden kann, wenn der Mikrorechner anhand der ihm zugeführten Daten erkennt, daß sich das Tonometer in der richtigen Position befindet - hierauf wird weiter hinten noch mit Bezug auf die Organisation des die Vorgänge des Tonometers steuernden Mikrorechners eingegangen.

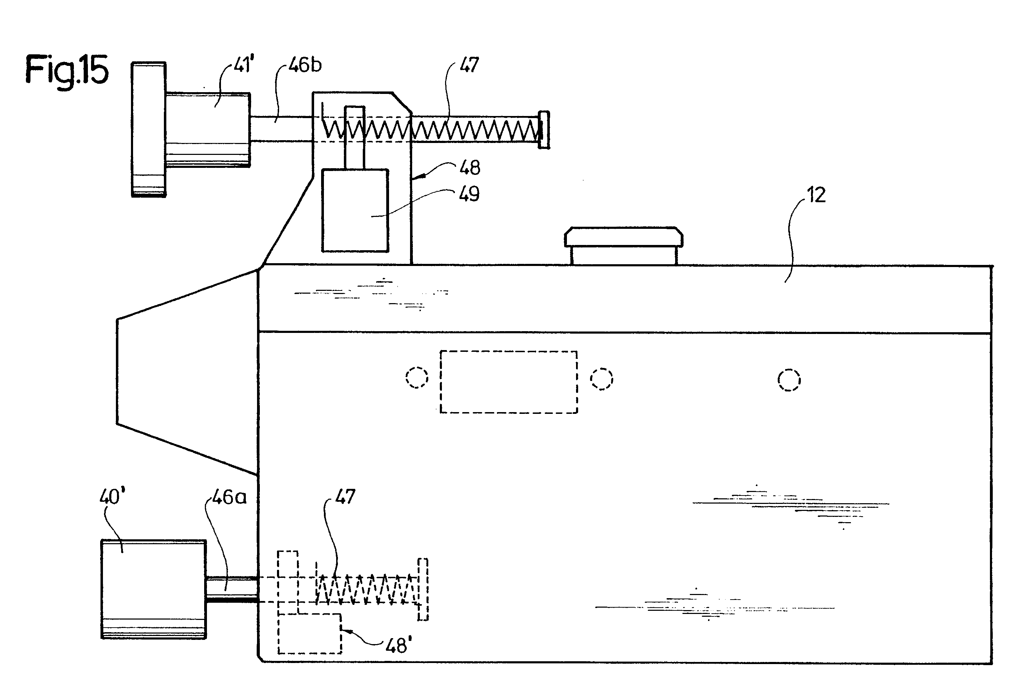

Eine alternative Ausführungsform einer ergänzenden Positioniereinrichtung, die ebenfalls vom Mikrorechner gesteuert werden kann, ist in Fig. 15 dargestellt. Bei diesem Ausführungsbeispiel sind die Wangenstütze 40' und die Stirnstütze 41' unabhängig voneinander am Gehäuse 12 des Geräts gelagert, und zwar über Lagerrohre 46a, 46b, die unter der Vorspannung weicher Zugfedern 47 in beispielsweise am Gehäuse 12 befestigten Lagerblöcken 48, 48' verschieblich gelagert sind. Diese Lagerblöcke enthalten gleichzeitig in geeigneter Weise vom Rechner aktivierbare und steuerbare Klemmvorrichtungen 49, so daß dann, wenn die vom Rechner erfaßte, neigungsfreie und ausgerichtete Position des Gerätes vor dem Auge erreicht ist, die Stirn- und Wangenstützen festgeklemmt werden, so daß daraufhin der Meßvorgang beginnen kann.

Bei diesen zusätzlichen Positioniereinrichtungen ist. daher wesentlich, daß beide Abstützungen für Stirn und Wange variabel gestaltet und durch eine geringe Federkraft so bewegt werden können, daß der gewünschte Abstand von Auge zu Gerät bzw. Meßeinrichtung beim Heranführen kleiner wird, wobei die Zugfedern 47 für die Anlage der Abstützungen am Gesicht des Probanden sorgen, bis die Endposition erreicht ist, woraufhin dann die Festklemmung erfolgt.

Ergänzend hierzu erlaubt im übrigen die in den Figuren 13 und 14 gezeigte Positionierabstützung eine Bewegung des Gerätegehäuses auf einer bestimmten, durch den Bügel vorgegebenen Bahn um das Auge bei Anliegen der die Stirn- und die Wangenstütze 40, 41 bildenden beiden Kontaktplatten 40" am Kopf. Die beiden Kontaktplatten sind dabei bevorzugt über Kugelgelenke 41" am Bügel gelagert. Das Nicken des Geräts um den Lagerpunkt als zusätzlichen Freiheitsgrad wird dann durch die Verschwenkbarkeit der Führungshülsen 44a, 44b gewährleistet.

Das Gerät verfügt ferner noch über eine Mehrzahl von Sicherheitseinrichtungen, die hauptsächlich dazu bestimmt sind, das Auftreten von für das menschliche Auge schädlichen Kräften beim Meßvorgang zu verhindern.

Eine erste Sicherheitseinrichtung kann dadurch hauptsächlich im Softwarebereich realisiert werden, daß über das PSD-Element 31' eine überhöhte Kraft durch das erfaßte Ausmaß der Meßfeder-Auslenkung erkannt und das entsprechende Reglersignal der PSD-LED für eine Überlasterkennung herangezogen wird, die dann darin bestehen kann, daß der vorzugsweise als Schrittmotor ausgebildete Antriebsmotor für den Schlitten 10 in seiner Drehrichtung reversiert wird.

Ferner ist ein spezieller Überlastschalter 50 (Fig.1) vorgesehen, der in Serienschaltung mit der Motoransteuerung den Motorstrom direkt abschaltet. Der Überlastschalter 50 ist so ausgebildet, daß er im Durchtrittsbereich einer der Lagerrohre für das Meßprisma 11, bei dem dargestellten Ausführungsbeispiel des unteren Lagerrohrs 26b, durch die entsprechende Bohrung 51 des hinteren Abschlußblocks 20' des Schlittens angeordnet ist; wird daher das Meßprisma 11 und damit auch dessen eines Lagerrohr 26b gegen die Wirkung der Meßfeder-Lagerung so weit zurückgedrückt, daß das hintere Rohrende den Stößel 50a des Überlastschalters 50 betätigen kann, dann wird der Motorstrom unmittelbar abgeschaltet.

Dabei können der Federweg (Schaltweg) sowie die zur Betätigung des Überlastschalters 50 erforderliche Federkraft als zusätzliche Rückmeldung bzw. Warnung an den Benutzer verwendet werden.

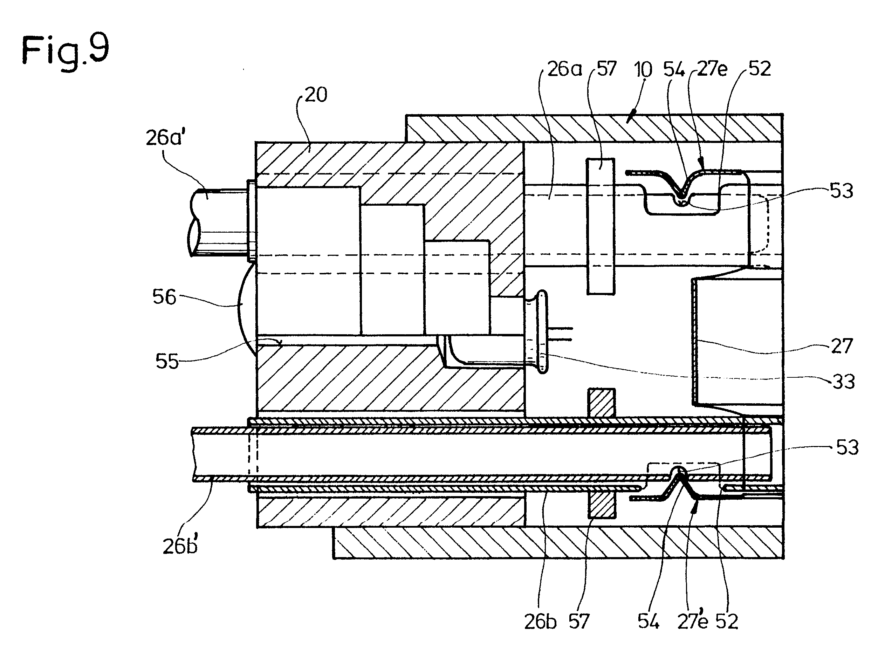

Eine weitere Sicherheitseinrichtung rein mechanischer Art besteht bei vorliegender Erfindung darin, daß die Rohrlagerung des Meßkörpers, also des Prismas 11 in Form einer Teleskopaufhängung ausgebildet ist, wie dies am besten der Darstellung der Fig. 9 erkennbar ist, die den Ausschnitt X der Fig. 1 genauer zeigt.

Beide Lagerrohre bestehen daher aus einem äußeren La gerrohrteil 26a, 26b, in welchem teleskopartig jeweils ein inneres Lagerrohr 26a', 26b' verschieblich gehalten und in einer vorgegebenen Position auch fixiert ist, so daß sich die beiden ineinander gesteckten Röhrchenpaare der Prismahalterung im Normalfall stets als Einheit relativ zum Schlitten 10 aufgrund ihrer federnden Lagerung verschieben können.

Dabei erfolgt die relative Fixierung der beiden Teleskopröhrchen zueinander mit Hilfe von vorgespannten Federn, die, wie Fig. 9 zeigt, durch eine Durchbrechung 52 im jeweils äußeren Röhrchen in eine Kerbe oder Ausnehmung 53 im inneren Röhrchen mit einem spitzen Vorsprung 54 eingreifen, wobei diese beiden Haltefederbereiche natürlich ortsfest am äußeren Röhrchen befestigt sind. Man erkennt, daß die in die Kerben 53 im inneren Röhrchen eingreifenden Federteile erst dann aus dieser Kerbe ausgehoben werden können, wenn die in Meßrichtung wirkende Kraft einen bestimmten Kraftschwellwert überschreitet, der als Sicherheitsschwellwert für den Benutzer des Geräts noch problemlos akzeptiert werden kann. In diesem Fall der Aushebung ergibt sich dann eine relativ leicht verschiebbare Teleskopanordnung der beiden Lagerröhrchenpaare ineinander, wodurch der Kraftschluß zwischen den beiden Röhrchen gelöst wird. In diesem Fall können die das Prisma 11 lagernden inneren Röhrchen mit dem Meßkörper bis in eine hintere Position verschoben werden.

Eine bevorzugte Ausgestaltung dieses Sicherungsbereichs besteht darin, daß die Arretierfedern ebenfalls Teil der Meßfeder 27 sind und seitlich von den Umschlingungsbereichen 32 ausgehen können, mit denen die Meßfeder an den äußeren Lagerröhrchen 26a, 26b befestigt ist. Daher sind die beiden Fixierfederbereiche, die die Relativposition der beiden ineinander teleskopierten Lagerröhrchen zueinander festlegen, auch mit 27e, 27e′ bezeichnet. Die Erfindung umfaßt noch eine Vielzahl weiterer Möglichkeiten zur Optimierung der Messung zunächst im mechanischen Bereich; beispielsweise kann der sich verjüngende vordere, das Meßprisma 11 umgebende und zum Teil einschließende Gehäusebereich 12a entweder selbst okularartig ausgebildet sein oder ein zusätzliches Okular tragen, welches so ausgebildet sein kann, daß es etwa nach Art von Film- oder Videokameras den äußeren Augenbereich vollständig umgibt, diesen abdeckt und an ihm anliegt, so daß hierdurch, gegebenenfalls unter Verzicht auf die Stirn- und Wangenabstützung der weiteren Positioniereinrichtung schon ein sicherer Halt für das Gehäuse des Tonometers erreicht wird. Dabei kann es vorteilhaft sein, ein solches Okular am Gehäuse 12 gelenkig und nach allen Richtungen beweglich zu lagern, wobei die freie Beweglichkeit dann wiederum, wie weiter vorn schon erläutert, durch eine entsprechende Ansteuerung vom Mikrorechner aus bei Erreichen der gewünschten Meßposition durch Festklemmen gesperrt werden kann.

Eine weitere vorteilhafte Ausgestaltung vorliegender Erfindung kann darin bestehen, daß man auch das Licht der Positionier-LED 33, welches durch eine sehr enge Bohrung 55 im Optikblock 20 nach vorn zum Meßprisma gelangt, zusätzlich in geeigneter Weise mit einer entsprechend dem gewünschten Bündelungsmaß ausgebildeten Linse 56 bündelt.

Der Darstellung der Fig. 9 läßt sich ferner noch ein stationär an den beiden Außenlagerrohren 26a, 26b angeordneter Anschlag 57 in Form jeweils von Anschlagringen entnehmen, der dazu dient, ein zu weites Herausziehen des Meßkörpers (Prisma 11) zu begrenzen, so daß die Lagerung durch die Meßfeder nicht überdehnt wird.

Diese Anschlagringe, stationär am jeweils äußeren Rohr befestigt, wirken mit einer weiteren mechanischen Sicherungseinrichtung zur Transportsicherung des Geräts zusammen, auf die im folgenden eingegangen wird. So ist zunächst noch ein Endpositionsschalter 52 vorgesehen, der durch den rückfahrenden Schlitten 10 in der Endposition elektrisch betätigt wird und durch entsprechende Ansteuerung über einen Eingang am Mikroprozessor dessen rückwärtige Endposition festlegt in Verbindung mit der Schrittmotoransteuerung. Mit diesem Endpositionsschalter 52 wirkt ein mechanischer, am Gehäuse stationär befestigter Endanschlag 53 zusammen, der in dieser erreichten Endposition an dem in der Darstellung der Fig. 1 oberen Lagerrohr 26a des Meßprismas 11, welches ebenfalls wie auch das untere Lagerrohr die Endplatte 20′ des Schlittens 10 durchsetzt, zur Anlage gelangt und hierdurch die beiden miteinander gekoppelten (äußeren) Lagerröhren 26a, 26b gerade so weit nach vorne schiebt, daß die vorderen Ringanschläge 57 (vgl. Fig. 9) am Optikblock 20 zur Anlage gelangen. Hierdurch ist der federnd und insoweit frei schwingend gelagerte und daher auch empfindliche Teil der Meßapparatur arretiert und für den Transport stabilisiert, so daß in dieser Anschlagposition beispielsweise auch Reinigungsarbeiten am Prisma durchgeführt werden können.

Um im übrigen das Prisma in seiner frei federnden Lagerung nicht zu weit nach innen schieben zu können, kann an den äußeren Lagerrohren 26a, 26b noch ein innerer Anschlag 54 befestigt sein, der an der hinteren Abschlußplatte 20′ innen dann zur Anlage kommt, wenn man das Prisma so weit nach innen drückt.

Der mechanische Aufbau des Geräteinneren des Tonometers vervollständigt sich dann noch hauptsächlich durch die elektronische Ausstattung einschließlich Mikroprozessor, wozu im freien Innenraum entsprechende Platinen und Schaltungsplatten 55 in nicht weiter zu beschreibender Weise angeordnet und gelagert sind. So stellt beispielsweise der Block 56 einen Spannungswandler dar; im unteren Teil des Gehäuses der Fig.1 befindet sich der Batteriekasten 57, wobei sich weiter nach links eine Sicherung 58 anschließt, die zum Hauptschalter 38 führt.

Die auf dem verfahrbaren Schlitten 10 angeordneten elektrischen und elektronischen Komponenten sind über ein flexibles Stromzuführungsband 59 mit den Hauptplatinen 55 verbunden.

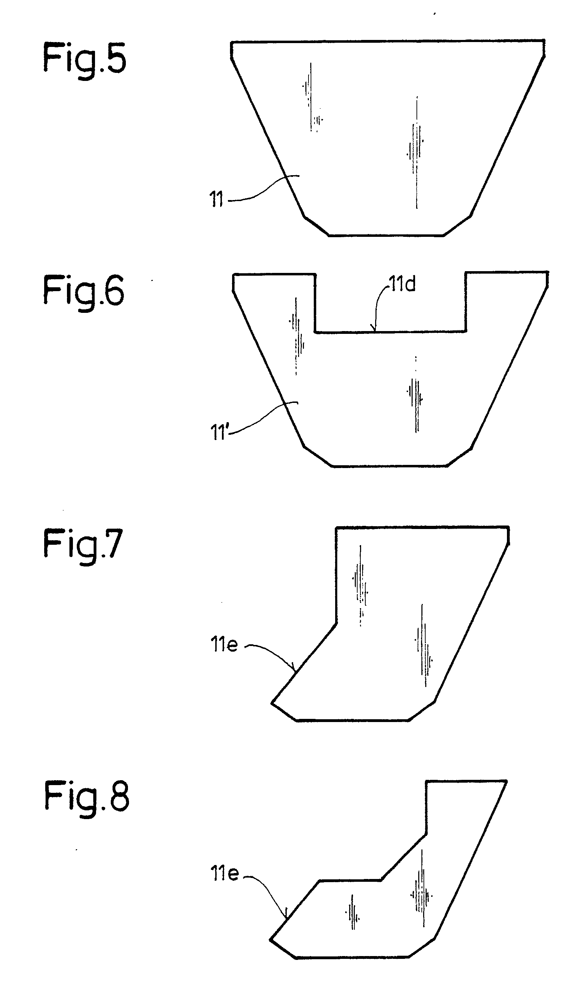

Entsprechend den Darstellungen in den Figuren 5, 6, 7 und 8 kann das Meßprisma beliebige Form annehmen, wobei die Grundform des Meßprismas in Fig. 5 dargestellt und mit 11 bezeichnet ist. Sämtliche Ausführungsformen verfügen über gebrochene Kanten, um die Positionierhilfe mittels der zusätzlichen Beleuchtungseinrichtung 37a, 37b zu gewährleisten; das Meßprisma 11′ der Fig. 6 weist den weiter vorn in Verbindung mit den Figuren 11 und 12 schon erwähnten hinteren Ausschnitt mit der inneren Rückfläche 11d auf, die die Ringstrukturen trägt; eine weitere Materialersparnis hinsichtlich der Prismakonfiguration ergibt sich bei den Ausführungsformen der Fig. 7 und 8, wobei die in der Zeichnung linken Prismateile weggelassen sind. Ein solcher Prismaaufbau bietet sich dann an, wenn man an der dann gebildeten Strahlenausgangswandung 11e unmittelbar einen entsprechend großflächigen Lichtempfänger anordnet und wie schon erwähnt dessen Zuleitungen über die Prismahalterung und die Federschenkel zum elektronischen Auswertebereich führt.

Eine weitere vorteilhafte Ausgestaltung der Erfindung besteht darin, unter Ausnutzung des von den Belichtungseinrichtungen 33, 37a, 37b erzeugten Lichts Abstands-Fotodioden anzusteuern, um so den Abstand Gerät/Auge zu bestimmen. Solche Abstands-Fotodioden 60a, 60b weisen schmale Öffnungswinkel auf und sind innen am sich verjüngenden Teil 12a des Gehäuses so angeordnet, daß nach dem Triangulationsverfahren der gewünschte Abstand bestimmt werden kann. Die Meßsignale der Abstandsfotodioden 60a, 60b gelangen ebenfalls zum Mikroprozessor,der diese bei der Einstellung der Positioniereinrichtungen (Wangenauflage; Stirnauflage) mitbenutzt. Dies kann beispielsweise so geschehen, daß zu Beginn der Messung das Gerät an das Auge herangefahren wird, wobei Stirn- und Wangenstütze 40', 41' - (vgl. Fig. 15) gegen den Druck ihrer weichen Vorspannungsfedern allmählich zurückgefahren werden, bis aufgrund der Triangulationsmessung mit den Abstands-Fotodioden der geeignete Abstand erreicht ist, woraufhin die Elektronik die Klemmeinrichtungen 49 betätigt.

Zusammengefaßt sind die Sicherheitsfunktionen daher so ausgelegt, daß eine auf das Auge einwirkende Kraft von beispielsweise 10 Pond niemals überschritten werden kann, weil

- bei Überdehnung der Meßfeder der Schlitten 10 softwaregesteuert zurückfährt, und zwar aufgrund des Ansprechens des PSD-Elements in Verbindung mit der Federauslenkungsbestimmung;
- dabei wird bei weiterer Dehnung der Meßfeder zusätzlich gegen die Kraft einer Kontaktfeder im Überlastschalter 50 der Motorstrom direkt unterbrochen - diese Funktion läuft also nicht über den Mikroprozessor, sondern ist unabhängig von dessen Funktionen und schließlich
- ist das Prisma mechanisch durch die Teleskopröhrenanordnung insofern gesichert, daß bei Überschreiten der Reibungskraft, mit welcher die Befestigungsfedern in den Kerben der inneren Röhrchen die Normallage des Prismas fixieren, die beiden Lagerrohrpaare sich zusammenschieben, wodurch sich das Meßprisma relativ zum Schlitten vom Auge wegbewegt.

Hierbei versteht es sich, daß die Ansprechschwelle des Überlastschalters 50 unter der der Teleskop-Sicherheitseinrichtung liegt, d.h. der Überlastschalter spricht auf jeden Fall zuerst an und erst dann, wenn die auf das Auge ausgeübte Kraft weiter ansteigen sollte, rutschen die Fixierfederteile 27e, 27e' aus den Kerben 53 und die Teleskoplagerung gibt in sich nach.

Ein wesentlicher Gesichtspunkt bei vorliegender Erfindung ist der durch die Zuordnung eines Mikrorechners mögliche vollautomatische Ablauf des jeweils vorgegebenen Meßalgorithmus bis zur Anzeige des Augeninnendrucks nach der Messung, in Verbindung mit sämtlichen Steuerfunktionen für die Mechanik, wie sie zum Teil weiter vorn schon angesprochen worden sind. Dabei ergibt sich ferner die Möglichkeit zur Durchführung von Reihenmessungen sowie die Abrufung der Daten über eine serielle Schnittstelle.

Mit Vorteil beruht die Grundfunktion vorliegender Erfindung darauf, durch die Aufnahme einer Vielzahl von in geeigneter Speicherform niedergelegter, einander zugeordneter Tabellenwerte von Druck (ausgeübter Kraft) und Applanationswert einen je nach Wunsch auch vollständigen Kennlinienverlauf einer sogenannten Applanationsfunktion zu erfassen und dann diese Kennlinie speziell in einem Bereich auszuwerten, der sich als besonders geeignet für die Messung des Augeninnendrucks herausgestellt hat, da sich in diesem die verschiedensten, insofern auch bekannten Einflußfaktoren mehr oder weniger gegenseitig aufheben oder herausgerechnet werden können. Ein solches Band von Applanationsdurchmessern kann beispielsweise im Bereich zwischen 2,5 bis 4 mm liegen, so daß entsprechend dem Diagrammverlauf der Fig. 4 nach der Aufnahme der gemessenen Werte, die sich als Kurvenverlauf I mit statistischen Schwankungen in entsprechender Abweichung nach oben oder unten um den tatsächlichen Kurvenverlauf II als Kennlinie des jeweils erreichten Applanationsdurchmessers bzw. der Fläche A von der Kraft F gruppieren, der Augeninnendruck aus der Steigung dF/dA ermittelt werden kann. Hierzu wird eine Ausgleichsgerade III an den durch die Tabellenwerte gegebenen Kurvenverlauf gelegt. Die ermittelte Tabelle ergibt darüber hinaus noch weitere Daten, nämlich entsprechend Fig. 4 einen Flächenoffset $A_O$, der eine Funktion des Applanationsdurchmessers sein kann und auf die Wirkung der Tränenflüssigkeit zurückzuführen ist, ferner einen Kraftoffset, der eventuell durch Lagefehler verursacht sein kann; die Erfindung ist in der Lage, einen solchen Lagefehler durch entsprechende Softwaregestaltung von Anfang an zu eliminieren, worauf noch eingegangen wird, wobei der Kraftoffset in der Rechnung keine Rolle spielt, wie aus der nachfolgenden Betrachtung hervorgeht. Durch die Bildung des Differenzenquotienten ergibt sich folgende Beziehung:

$$p = \Delta F/\Delta A = \frac{(F_4+F_O) - (F_3+F_O)}{A_4-A_{tr4}-(A_3-A_{tr3})}$$

$$= \frac{F_4-F_3}{A_4-A_3-(A_{tr4}-A_{tr3})}$$

Dabei bezeichnen die Indices 3 bzw. 4 die Werte von Fläche A und Kraft F bei einem Applanationsdurchmesser von 3 bzw. 4 mm - mit $A_{tr3}$ bzw. $A_{tr4}$ ist die durch den Tränenflüssigkeitsfilm verursachte Fläche bezeichnet bei diesen Durchmessern.

Untersucht man die Änderung der Fläche des Tränenflüssigkeitsfilms im betrachteten Bereich, wenn die Volumenkonstanz der Tränenflüssigkeit, wie sicher gerechtfertigt für die sehr kurze Meßzeitdauer, vorausgesetzt wird, dann ergeben sich durch überschlägige Rechnungen, daß eine Änderung der Tränenflüssigkeit im betrachteten Bereich so gut wie nicht vorliegt. Dies kann darauf zurückzuführen sein, daß sich bei größeren Applanationsdurchmessern ein dünnerer Tränenflüssigkeitsring ergibt, da sich in diesem Fall das Flüssigkeitsvolumen über einen größeren Umfang verteilen muß. Das bedeutet, daß bei den im folgenden noch genauer zu erläuternden Meßvorgängen entsprechend vorliegender Erfindung eine Ermittlung des Tränenflüssigkeitsfilms entbehrlich ist, jedenfalls dann, wenn man die gewünschten und durch Messung zu ermittelnden Tabellenwerte von Fläche und Kraft in den Applanationsdurchmesserbereich zwischen 3 bis 4 mm legt, da in diesem Bereich der Flüssigkeitsring eine Breite von zwischen 0,1 bis 0,4 mm hat und sich entspresprechend der Formel die Tränenflüssigkeitsflächen herausheben, wobei sich die Tränenflüssigkeitsflächen in etwa konstant verhalten.

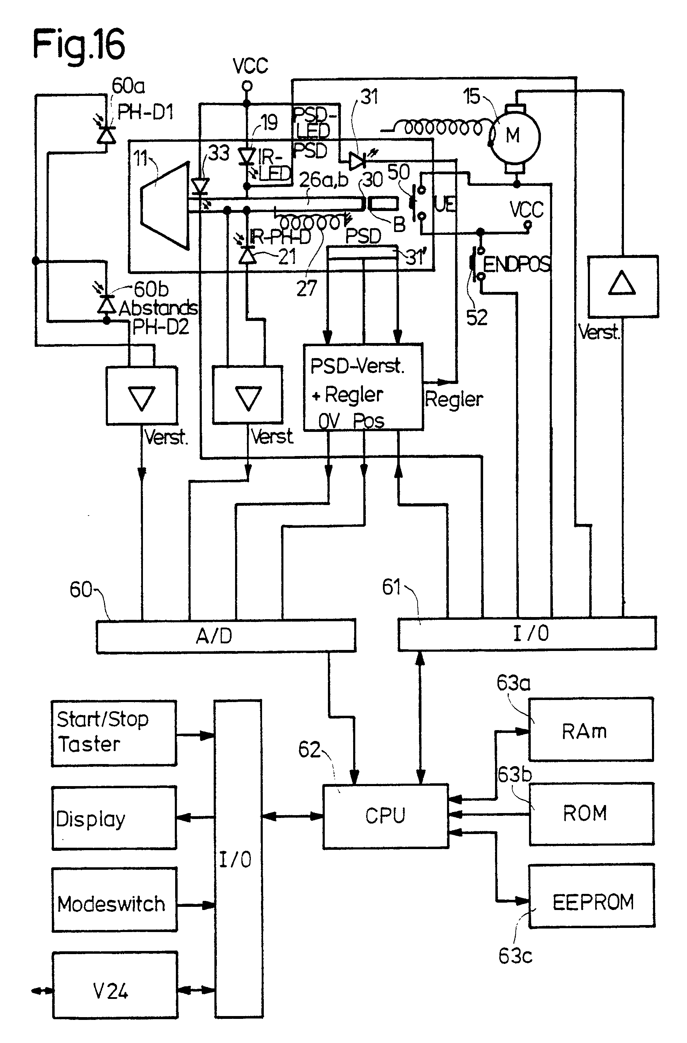

Eine mögliche Ausführungsform der Mikrorechner/Eingangs- und Ausgangsanschlüsse, seine Organisation und der Zuordnung der äußeren Schaltungselemente ist in Fig. 16 dargestellt, wobei zur Erleichterung der Gewinnung eines Überblicks zusätzlich zu den Eintragungen auch noch die Bezugszeichen für die einzelnen Elemente angegeben sind.

Im reinen Eingangsbereich entsprechend Eingangs-Analog/Digitalwandler 60 liegen die Abstandssensor dioden 60a, 60b, der IR-Sensor für die Flächenmessung, umfassend die IR-LED 19 und die IR-Fotodiode 21 (vgl. auch Fig. 10), ferner die Eingangsdaten für die Bereichsüberschreitung (overload-signal) sowie Positionssignal vom PSD-Element 31'.

An den anderen Eingangs/Ausgangsbereich (I/O-Interface 61) sind dann die restlichen Signaleingänge bzw. -ausgänge angeschlossen einschließlich der Motoransteuerung. Dabei sind der CPU-Einheit 62 des Mikrorechners drei verschiedene Speicherbereiche zugeordnet, nämlich ein RAM-Speicher 63a, ein ROM-Speicher 63b sowie ein EEPROM-Speicherbereich 63c. Hier sind die längerfristigen Daten niedergelegt, die der Mikrorechner braucht, um letztendlich aus den eingehenden gemessenen Tabellenwerten den Augeninnendruck zu berechnen.

Dieser Augeninnendruck wird zwar aufgrund der weiter vorn angegebenen Formel aus der sogenannten Ausgleichsgeraden entsprechend Kurvenverlauf III in Fig. 4 ermittelt; diese Ausgleichsgerade ist aber das Produkt aus sämtlichen vorhergehenden Einzelmessungen, die dem um den Mittelwert des Kurvenverlaufs II schwankenden Meßwertsverlauf I zugrunde liegen, wobei unter Zugrundelegung aller ermittelten Einzelmeßwerte die Ausgleichsgerade bzw. deren Steigung errechnet wird. Dies bedeutet im übrigen mit anderen Worten, daß bei den jeweiligen Einzelmessungen natürlich auch alle Störeinflüsse und peripheren Fehler in das jeweilige Meßergebnis eingehen, die sich jedoch statistisch gesehen unter Zugrundelegung eines entsprechenden Meßalgorithmus im Mikrorechner praktisch herausmitteln, so daß es durch die Erfindung erstmals gelingt, ein Meßergebnis zur Verfügung zu stellen, welches präzise dem Augeninnendruck entspricht und nicht aus einer jeweiligen Einzelmessung, die notwendigerweise immer mit irgend welchen, zum Teil gar nicht erfaßbaren Fehlergrößen behaftet ist, resultiert.

Die Aufteilung der Speicher kann dann so getroffen sein, daß im ROM 63b das komplette Programm einschließlich des Meßalgorithmus enthalten ist, während im RAM-Speicher 63a beliebige Arbeitsdaten, auch kurzzeitiger Natur enthalten sind; beispielsweise kurzfristig berechnete Daten wie Kraftoffset aus dem PSD-Bereich (hierauf wird weiter unten noch eingegangen), Zwischenspeicherung der aufgenommenen Tabellenwerte; während im EE-PROM-Speicher 63c Kennliniendaten etwa über das Verhalten des PSD-Elements zur Linearisierungskorrektur enthalten sein können, der Wert der Federkonstanten der Meßfeder, Verhalten der Feder bei Auslenkung, welches wegen der Parallelogrammführung notwendigerweise einer wenn auch nur sehr geringen Kreisbogenführung entspricht.

Im folgenden wird schließlich noch ein möglicher Betriebsablauf eines Meßvorgangs mit dem erfindungsgemäßen Tonometer so wie sich dieser im Bereich des Mikrorechners ergibt, anhand eines Flußdiagramms zur umfassenden Information dargestellt, wobei aber darauf hingewiesen wird, daß die dort angegebenen einzelnen Funktionsblöcke lediglich eine mögliche Ausführungsform darstellen und, wie im übrigen auch die weiter vorne angegebene Beschreibung des bevorzugten Ausführungsbeispiels die Erfindung nicht einschränken.

Start     I

Gerät einschalten     II

RAMTEST     III

Warmstart     IV

Schlitten nach vorn verfahren     IV'

Ermittlung der Periodendauer der Schwingung des Meßkörpers
Zeitbasis: = Periodendauer/n     V

Schlitten zurück bis zum Betätigen des Endschalters     VI

keine sinnvolle Zeit gefunden?     VII

Nein

Defaultwert nehmen

Ja

auf Tasterimpuls warten 0.2 sec<t<1 sec     VIII

Positionier-LED einschalten     IX

Auf START/STOP-Taster warten     X

Positionier-LED 2 x blinken lassen     XI

4 sec warten

IR-LED einschalten   XII

100 % Sensorwert ermitteln   XIII

Applanationsschwelle
festlegen   XIV

Schlitten nach vorne fahren   XV

Applanationsschwelle
unterschritten?   XVI

Ja

Nein

Grenzwert überschritten?   XVII

Ja

Nein

Schlitten zurück

Warten auf Zeitbasis   XVIII

RESET

XIX

ein Wert von der Kraftmeßeinrichtung
lesen und aufsummieren bis
n Werte gesammelt sind
dann diesen Wert in einen Ringbuffer schreiben

14

```
                        ┌─────────────────────┐
                        │   0.5 sec warten    │  XX
                        └─────────────────────┘
                                   │
                        ┌─────────────────────┐
                        │ ältesten Kraftwert aus │
                        │ dem Ringbuffer nehmen  │  XXI
                        │   - > Kraftoffset     │
                        └─────────────────────┘
                                   │
                        ┌─────────────────────┐
                        │ die momentan ausgeübte Kraft │  XXII
                        │    auf Null ausregeln │
                        └─────────────────────┘
                                   │
                        ┌─────────────────────┐
                        │ Tränenflüssigkeit in diesem │
                        │ Zustand mit dem Sensor │  XXIII
                        │  messen und speichern │
                        └─────────────────────┘
                                   │
                        ┌─────────────────────┐
                        │ Schlitten sehr langsam │  XXIV
                        │   nach vorne fahren  │
                        └─────────────────────┘
                                   │
                        ┌─────────────────────┐
                        │  auf Zeitbasis warten │  XXV
                        └─────────────────────┘
                                   │
                        ┌─────────────────────┐
                        │    Kraftwerte in     │
                        │   Tabelle eintragen  │
                        │ Applanierte Fläche - > Tabposition │  XXVI
                        │ Kraftwert - > Funktionswert │
                        └─────────────────────┘
                                   │
                             XXVII ◇ Grenzwert erreicht?  ──Ja──┐
                                   │ N                          │
                            XXVIII ◇ Appl. Fläche >            ┌──────────────┐
                      Nein ────────  Amax. ?                   │ Schlitten zurück │
                                   │ Ja                        └──────────────┘
                        ┌─────────────────────┐                      │
                        │  Schlitten zurück   │                  ( RESET )
                        └─────────────────────┘
                                   │
                        ┌─────────────────────┐
                        │  Positionier-LED    │
                        │  blinken lassen     │
                        └─────────────────────┘
                                   │
                        ┌─────────────────────┐
                        │  Ergebnis berechnen  │
                        │    und anzeigen      │
                        └─────────────────────┘
                                   │
                             ( Warmstart )
```

Auf die fünf ersten Initialisierungsblöcke I von "Start" bis V entsprechend der Zurückfahrt des Schlittens bis zur Betätigung des Endschalters braucht nicht eingegangen zu werden; der sich daran anschließende Funktionsblock VI ist insofern interessant, als dieser darauf gerichtet ist, die Periodendauer der Schwingung des Meßkörpers im Ausgangszustand zu ermitteln; eine Schwingung, die auch während der Messung auftritt und mindestens mitverantwortlich ist bzw. die Zeitbasis vorgibt für den statistisch um die eigentlich gewünschte Kennlinie II nach oben und unten abweichenden Schwingungsverlauf der tatsächlichen Meßwertgewinnung entsprechend Kennlinie I. Ist vom Mikrorechner diese Grundschwingung der Periodendauer festgestellt, dann ergibt sich hieraus eine Zeitbasis und die Messung läuft dann so ab, daß man in dieser Zeitbasis, was einer einzigen Schwingung des Kennlinienverlaufs I entspricht, eine vorgegebene Anzahl von Einzelmessungen unterbringt, beispielsweise und um hier eine die Erfindung natürlich nicht einschränkende numerische Zahl zu nennen, 16 Messungen während einer Schwingung. Auf diese Weise gelingt es nämlich, so viele Daten über den Kennlinienverlauf zu sammeln, daß dann sinnvoll über den Bereich im Kennlinienverlauf II entsprechend Fig. 4 schließlich die Ausgleichsgerade I in ihrer Steigung ermittelt werden kann.

Ist entsprechend nachfolgendem Schaltungsblock VII keine sinnvolle Zeitbasis gefunden worden, dann wird aus einem der Speicher, vorzugsweise dem EE-PROM 63c ein Default-Wert genommen, der auf der Erfahrung früherer Messungen beruht.

Anschließend wird abgewartet, bis die Bedienungsperson den Start/Stop-Taster 39 betätigt, woraufhin die Positionier-LED 33 eingeschaltet wird. Wird dann der Start/Stop-Taster durchgehend niedergedrückt gehalten, dann wird diese entsprechend Funktionsblock XI zum Blinken gebracht; es wird dann wieder abgewartet und anschließend die für die Bestimmung des Flächenwerts zuständige IR-LED 19 eingeschaltet (Funktionsblock XII). Anschließend wird der 100 %-Sensorwert ermittelt, d.h. die Lichtmenge, die auf das PSD-Element 31' dann auffällt, wenn am Meßprisma 11 noch Totalreflexion vorliegt, dieses also das Auge nicht berührt hat (Funktionsblock XIII), woraufhin dann eine Applanationsschwelle festgelegt wird (Funktionsblock XIV), die beispielsweise bei 97 % des 100 %igen Sensorwerts liegen kann.

Anschließend geht das System, und zwar vor dem Beginn der eigentlichen Messung in eine Warteschleife, gebildet durch die Funktionsblöcke XV bis XIX und zurück zum Funktionsblock XV "Schlitten nach vorn fahren", bis die vorgegebene Applanationsschwelle unterschritten ist, d.h. bis ein Kontakt am Meßprisma mit dem Auge stattgefunden hat.

In dieser Warteschleife können Grenzwerte überschritten werden, die eine Zurücksetzung des Schlittens erforderlich machen, beispielsweise also Ansprechen des Überlastschalters, Überdehnung des PSD-Elements u. dgl. In dieser Warteschleife werden ferner schon, nachdem die Zeitbasis vorgegeben ist (Funktionsblock XVIII), Werte der Kraftmeßeinrichtung (PSD-Element 31') gelesen und aufsummiert und in einen Speicher eingeschrieben. Dies dient der Nullpunktbestimmung des Kraftsensors (PSD), wobei eine Vielzahl solcher Werte gesammelt und in einen Ringbuffer eingeschrieben werden. Durch diese Nullpunktbestimmung lassen sich auch die Folgen einer gewissen Schräghaltung des Geräts ausgleichen, denn tatsächlich bedeutet eine Neigung des Geräts, daß sich eine Verlagerung des Meßprismas schon allein aufgrund der herrschenden Anziehungskräfte ergibt.

Sobald dann die Applanationsschwelle effektiv unterschritten ist, wird bei einer vorgegebenen Wartezeit (Funktionsblock XX) an den folgenden Funktionsblöcken XXI, XXII und XXIII der Einfluß der Tränenflüssigkeit zusätzlich ermittelt und abgespeichert, obwohl dies für den eigentlichen Meßvorgang, wie weiter vorn anhand der Formeln schon erläutert, nicht erforderlich ist.

Erst daran schließt sich dann der tatsächliche Meßvorgang mit dem Funktionsblock XXIV (langsame Nachvornebewegung des Schlittens) an, der nach Zuführung der Zeitbasis (Funktionsblock XXV) im Funktionsblock XXVI dann dazu führt, daß die jeweils von der Kraftmeßeinrichtung ermittelten Kraftwerte in die Tabelle eingetragen werden, wobei die hierzu gleichzeitig ermittelten Flächenwerte beispielsweise als Adressen dienen können. Wird während dieser Messung ein Kraft-Grenzwert erreicht (Funktionsblock XXVII), dann wird der Schlitten zurückgefahren und es kommt zum Reset; ist dann entsprechend dem nachfolgenden Funktions block XXVIII der angestrebte Maximalwert der applanierten Fläche erreicht, dann fährt der Schlitten zurück und das Ergebnis wird berechnet. Andernfalls fährt der Schlitten weiter nach vorn und die Tabellenwerte werden vervollständigt.

Da entsprechend dem Funktionsblock XXII im Moment des Überschreitens der Applanationsschwelle die momentan ausgeübte Kraft auf Null ausgeregelt wird, ergibt sich entsprechend dem Diagrammverlauf der Fig. 4 der bei der Kraft $F = 0$ auf die Tränenflüssigkeit zurückzuführende Flächenoffset $A_0$, bei welchem Wert die Flächenmessung beispielsweise je nach dem zugrunde gelegten Meßalgorithmus beginnen kann, wobei es aber auch möglich und sinnvoll ist, lediglich die Werte im Bereich zwischen den Applanationsdurchmessern 3 mm bis 4 mm aufzunehmen, da, wie weiter vorn schon erläutert, hier die wenigsten Störfaktoren vorliegen bzw. sich gegenseitig aufheben.

Eine alternative Ausgestaltung bei vorliegender Erfindung besteht noch darin, daß man in geeigneter Weise die Schwingungsfähigkeit des federgelagerten Systems dämpft, etwa indem man eine Luftkammer anordnet, in welcher sich ein Flügel der Meßfeder bewegt oder daß man die Meßfeder selbst in einen Luftkammerbereich einschließt; es ist aber auch möglich, eine Dämpfung in Form einer Wirbelstrombremse vorzusehen, beispielsweise durch die Zuordnung eines festen Dauermagneten zu einem beweglichen Blechteil, welches mit dem Meßsystem verfährt.

Bei der federelastischen Lagerung des Meßkörpers ist ferner noch von Bedeutung, daß für das Gewicht des Meßkörpers und der Feder keine Ausgleichsmasse erforderlich ist, da die erforderliche Lageunabhängigkeit im Softwarebereich erreicht wird, indem während des Schwingungsverhaltens des Systems der Nullwert aufgesucht und festgelegt wird. Ferner ist es möglich, durch eine entsprechende Beschaltung mögliche Alterungserscheinungen im PSD-Bereich oder Empfindlichkeitsveränderungen zu minimieren, indem man Summenschaltungen des gesamten, vom PSD-Element abgegebenen Stroms mit einem Sollwert vergleicht und entsprechend die Helligkeit der IR-Leuchtdiode 31 nachregelt.

Ferner ergibt sich ein weiterer Vorteil im Bereich der Meßfeder dadurch, daß diese auch gewisse Auslenkungen senkrecht zur Meßrichtung ermöglicht, ohne daß das Meßergebnis beeinflußt wird. Dies erreicht man durch den symmetrischen Aufbau der Meßfeder, die, wie weiter vorn schon erläutert, mit ihren beiden Hauptschenkeln 27a, 27b bis nahe zum PSD-Element 31' reicht und dann mit Lagerlappen 27d, 27e zurückgeführt ist bis zu den Befestigungspunkten an den Lagerrohren 26a, 26b. Man erkennt hierdurch, daß zwischen der Meßfläche und der Augenoberfläche auftretende mögliche Scherkräfte, die insofern Auslenkungen senkrecht zur Meßrichtung sind, tolerierbar bzw. zulässig werden, weil die rückgeklappten Lagerlappen 27d, 27e der Meßfeder solche Bewegungen längs des in Fig.3 angezeigten Doppelpfeils C ermöglichen, ohne daß hierdurch die Meßgenauigkeit leidet.

Alle in der Beschreibung, den nachfolgenden Ansprüchen und der Zeichnung dargestellten Merkmale können sowohl einzeln als auch in beliebiger Kombination miteinander erfindungswesentlich sein.

## Ansprüche

1. Verfahren zur Bestimmung des Augeninnendrucks oder ähnlich empfindlicher Zusammenhänge zwischen einer applanierten Fläche und dem für die Herbeiführung der Applanation erforderlichen Druck, wobei ein Meßkörper (Prisma) mit vorgegebener Kraft auf das menschliche Auge aufgebracht und die entsprechend applanierte Fläche mittels eines sich hierdurch ändernden Lichtstreuungsverhältnisses bestimmt und ferner aus dieser gemessenen Fläche und der ebenfalls gemessenen Kraft der Augeninnendruck (als Quotient) abgeleitet wird, dadurch gekennzeichnet, daß im Verlauf einer kontinuierlichen Druckkrafterhöhung eine Vielzahl von jeweils zueinander gehörenden Meßwerten von Kraft und applanierter Fläche ermittelt, in Tabellenform der so erfaßte Kurvenverlauf (Kennlinie) niedergelegt und aus den aufgenommenen Meßwerten der Augeninnendruck differentiell ermittelt wird.

2. Verfahren nach Anspruch 1, dadurch gekennzeichnet, daß zur verfahrwegunabhängigen Gewinnung des Flächenmeßwerts die zur Reflexion auf das Meßprisma (11) geworfene Strahlung parallel gebündelt wird.

3. Verfahren nach Anspruch 1 oder 2, dadurch gekennzeichnet, daß zur positionsunabhängigen Gewinnung des Kraftmeßwerts die im Moment des Erreichens der Applanationsschwelle ausgeübte Kraft durch Regelung der Schlittenposition auf Null ausgeregelt wird.

4. Verfahren nach einem der Ansprüche 1-3, dadurch gekennzeichnet, daß zur Kraftmessung ein optischer Sensor über eine Blende, der Auslenkung der federnden Meßprismaaufhängung folgend, angestrahlt, der Verschiebeweg erfaßt und unter Einbeziehung der Federkonstanten in zur jeweiligen Applanationsfläche korrelierte Kraftwerte umgesetzt wird.

5. Verfahren nach einem der Ansprüche 1-4, dadurch gekennzeichnet, daß vor Beginn jeder Messung die Periodendauer der Schwingung des Meßkörpers (Meßprisma 11) erfaßt und eine Korrelation zwischen Schwingung des Prismas und Anzahl der Einzelmessungen hergestellt wird.

6. Verfahren nach einem der Ansprüche 1-5, dadurch gekennzeichnet, daß die Messungen ohne Berücksichtigung der auf den Einfluß der Tränenflüssigkeit zurückzuführenden Applanationsfläche (Flächenoffset $A_o$) über die gesamte Kennlinie beginnend mit dem Kraftwert Null oder lediglich über einen vorgegebenen Kennlinienzwischenbereich durchgeführt werden.

7. Verfahren nach Anspruch 6, dadurch gekennzeichnet, daß der gewählte Zwischenbereich der Kennlinienbestimmung im Bereich zwischen ca. 3 mm Applanationsdurchmesser bis ca. 4mm Applanationsdurchmesser liegt.

8. Verfahren nach einem der Ansprüche 1-7, dadurch gekennzeichnet, daß unter Zugrundelegung des durch Vielfachmessung bestimmten Kennlinienabschnitts eine Ausgleichsgerade im Sinne einer besonderen Regression ermittelt und aus deren Steigung auf den zu bestimmenden Augeninnendruck geschlossen oder durch eine aus der Statistik bekannte Medianermittlung die Steigung erfaßt wird, wobei in beiden Fällen der Berechnung entweder das Verhältnis aus Kraft zu Fläche (F/A) in Abhängigkeit der Zeit oder die jeweilige Kraft (F) in Abhängigkeit der Fläche (A) zugrundegelegt ist.

9. Verfahren nach einem der Ansprüche 1-8, dadurch gekennzeichnet, daß zu Beginn jeder Messung nach Einschalten des Lichtsenders (IR-LED 19) für die Flächenbestimmung der 100 %-Wert bei Vollreflexion ermittelt und aus diesem eine knapp unterhalb dieses maximalen Reflexionswertes liegende Applanationsschwelle festgelegt wird, bei deren Unterschreiten der Meßvorgang durch allmähliches Heranfahren eines sowohl die Kraftmeßeinrichtung als auch die Flächenmeßeinrichtung tragenden, motorisch verschiebbaren Schlittens an das Auge eingeleitet wird.

10. Verfahren nach einem der Ansprüche 1-9, dadurch gekennzeichnet, daß vor Erreichen der Applanationsschwelle beim allmählichen Heranfahren an das Auge in einer Warteschleife die Nullpunktbestimmung des Kraftsensors durchgeführt wird, indem von der Kraftmeßeinrichtung gelieferte Werte gelesen und in einen Ringspeicher eingeschrieben werden.

11. Verfahren nach einem der Ansprüche 1-10, dadurch gekennzeichnet, daß bei Überdehnung der den Meßkörper (Prisma 11) gleichzeitig federnd lagernden und durch ihre Auslenkung den jeweils ausgeübten Kraftwert durch optische Wegkraftmessung liefernden Meßfeder der das Meßsystem lagernde Schlitten softwaregesteuert zurückgefahren wird.

12. Verfahren nach einem der Ansprüche 1-11, dadurch gekennzeichnet, daß bei weiterer Dehnung der Meßfeder zusätzlich die Kraft einer Kontaktfeder (in einem Überlastschalter) überwunden und der Motorstrom zum Antrieb des Schlittens unterbrochen und dem Microcontroller mitgeteilt wird.

13. Verfahren nach einem der Ansprüche 1-12, dadurch gekennzeichnet, daß sich bei Auftreten einer über der Ansprechkraft der Kontaktfedern im Überlastschalter liegenden, letztlich vom Meßprisma ausgeübten Kraft in der Halterung des Meßprismas angeordnete Teleskoprohre ineinanderschieben derart, daß sich das Meßprisma relativ zum Schlitten vom Auge wegbewegt.

14. Verfahren nach einem oder mehreren der Ansprüche 1-13, dadurch gekennzeichnet, daß zu Beginn des Meßvorgangs eine der Positionierung dienende Beleuchtungsquelle (Leuchtdiode 31) aktiviert wird, die eine Grundstruktur im Meßprisma (11) dann voll ausleuchtet, wenn sich Lichtquelle, Prisma und die Cornea des Auges neigungsfrei auf einer Achse befinden.

15. Vorrichtung zur Bestimmung des Augeninnendrucks oder ähnlich empfindlicher Zusammenhänge zwischen einer applanierten Fläche und dem für die Herbeiführung der Applanation erforderlichen Druck, wobei ein Meßkörper (Prisma) mit vorgegebener Kraft auf das menschliche Auge aufgebracht und die entsprechend applanierte Fläche mittels eines sich hierdurch ändernden Lichtstreuungsverhältnisses bestimmt und ferner aus dieser gemessenen Fläche und der ebenfalls gemessenen Kraft der Augeninnendruck (als Quotient) abgeleitet wird, zur Durchführung des Verfahrens nach einem oder mehreren der Ansprüche 1-14, dadurch gekennzeichnet, daß

a) ein motorisch verfahrbarer Schlitten (10) vorgesehen ist, daß

b) auf dem Schlitten (10) der Meßkörper (Prisma 11) reibungsfrei und seinerseits zum Schlitten relativ verschieblich gelagert ist, daß

c) eine die Relativverschiebung zwischen Schlitten (10) und Meßkörper (Prisma 11) erfassende Meßeinrichtung (30, 31, 31') vorgesehen ist, die diese Relativverschiebung in Kraftwerte umgesetzt definiert und daß

d) eine optische, das Meßprisma (11) umfassende Erfassungseinrichtung für die durch die Bewegung des Meßkörpers zunehmend applanierte Fläche vorgesehen ist, wobei ferner Verrechnungsmittel (Mikrorechner) vorgesehen sind, die die ermittelten Kraftwerte mit den Flächenwerten korrelieren und daraus den Augeninnendruck bestimmen.

16. Vorrichtung nach Anspruch 15, dadurch gekennzeich net, daß der Meßkörper ein Prisma (11) beliebiger, auch lediglich einseitiger Form (Fig. 5, Fig. 6, Fig. 7, Fig. 8) ist und von einer Meßfeder (27) am Schlitten (10) federnd nachgiebig gehalten ist, wobei die sich bei Schlittenvorschub und Anlage des Prismas (11) am Auge ergebende Federauslenkung von der Meßeinrichtung (30, 31, 31') erfaßt ist.

17. Vorrichtung nach Anspruch 16, dadurch gekennzeichnet, daß die Meßeinrichtung (30, 31, 31') zur Erfassung der Federauslenkung aus einem Beleuchtungskörper (PSD-Leuchtdiode 31), einer mit der Auslenkung verschiebbaren Schlitzblende (30) und einem PSD-Empfängerelement (31) besteht.

18. Vorrichtung nach einem der Ansprüche 15-17, dadurch gekennzeichnet, daß die das Meßprisma (11) lagernde Meßfeder (27) aus zwei zueinander parallelen, eine Art Parallelogrammführung bildenden Hauptschenkeln (27a, 27b) besteht, die am Schlitten (10) befestigt sich in Querrichtung zur Schlittenverschieberichtung erstrecken und in einen die Schlitzblende lagernden Hauptquersteg (27c) übergehen, von welchem

18

EP 0 418 746 A1

nach rückwärts in Richtung der beiden Hauptschenkel verlaufend Lagerlappen (27d, 27e) ausgehen, die ein den Meßkörper nach vorn lagerndes Rohrgestänge (26a, 26a'; 26b, 26b') tragen.

19. Vorrichtung nach Anspruch 18, dadurch gekennzeichnet, daß als eine zuletzt ansprechende Sicherheitsvorrichtung das den Meßkörper lagernde Rohrgestänge jeweils zwei Trägerrohre (26a, 26a'; 26b, 26b') umfaßt, die ineinander teleskopierbar und unter Federdruck in einer auseinandergezogenen Normalposition gehalten sind.

20. Vorrichtung nach Anspruch 19, dadurch gekennzeichnet, daß jeweils ein Federvorsprung (54) durch eine Ausnehmung (52) im äußeren Tragrohr in eine Kerbe (53) im inneren Tragrohr eingreift und so die beiden ineinander teleskopierbaren Tragrohrteile zueinander mit vorgegebener Schwellenkraft fixiert, wobei die Federvorsprünge (54) Teil der Meßfeder (27) sind und von den die äußeren Tragrohre (26a, 26b) jeweils umfassenden Trägerlappen (27d, 27e) ausgehen.

21. Vorrichtung nach einem der Ansprüche 1-20, dadurch gekennzeichnet, daß der im Gehäuse längs einer Führungsschiene (13) von vorzugsweise schwalbenschwanzähnlicher Konfiguration gleitverschieblich gelagerte Schlitten (10) mit einer Kupplungsfeder (18) in eine Antriebsspindel (16) des Antriebs-Schrittmotors (15) eingreift und auf seiner einen Längsseite einen Lagerblock (28) für die Fixierung der beiden Federhauptschenkel (27a, 27b) und zur Aufnahme der PSD-Leuchtdiode (31) lagert, während auf der gegenüberliegenden Seite und angrenzend zur von dem Federbasisquersteg (27c) gebildeten Meßblende (30) das sich in Schlittenverschieberichtung erstreckende PSD-Widerstandselement (31') angeordnet ist.

22. Vorrichtung nach einem der Ansprüche 15-21, dadurch gekennzeichnet, daß der Schlitten (10) in seinem in Verschieberichtung gesehen vorderen Teil einen Optikblock (20) lagert, der neben den IR-Leucht- und Fotodioden (19, 21) für die Bestimmung der Applanationsfläche zentral eine Positionier-Leuchtdiode (33) lagert sowie die Bündelungslinsen (22, 25) für die von den Flächenmeßelementen ausgehenden Lichtstrahlen, wobei der Optikblock (20) ferner berührungsfrei durchsetzt ist von den in der Höhe zueinander versetzten Lagerrohren (26a, 26b; 26a', 26b') für das Meßprisma.

23. Vorrichtung nach Anspruch 22, dadurch gekennzeichnet, daß innen im Bereich einer sich nach vorn verjüngenden Gehäusewandung (12a) weitere Beleuchtungskörper (37a, 37b) als Positionierhilfen vorgesehen sind und daß zur Grob- und Feinpositionierung der Block des Meßprismas (11) an seinen Seitenflächen und im hinteren, von vorne erkennbaren Abschlußflächen geschwärzt ist mit einer auf die zentrale Positionier-Beleuchtungseinrichtung (Positionier-Leuchtdiode 33) ausgerichteten Struktur, vorzugsweise in Form zweier transparenter, zueinander konzentrischer Kreise (35a, 35b) sowie einer durch gebrochene Kanten erzeugten Flächenumrandung.

24. Vorrichtung nach einem der Ansprüche 15-23, dadurch gekennzeichnet, daß weitere Positionierhilfen in Form einer variable Stirn- und Wangenstützen (40', 41') umfassenden Kopfabstützung vorgesehen sind.

25. Vorrichtung nach Anspruch 24, dadurch gekennzeich net, daß Stirn- und Wangenstützen (40', 41') an einem kippbaren und in Höhe verschiebbaren, vorzugsweise umlaufenden Bügel über Kugelgelenke (41) befestigt sind, mit Mitteln zur manuellen oder vom Rechner gesteuerten Festklemmung der Position von Stirn- und Wangenstütze.

26. Vorrichtung nach Anspruch 24, dadurch gekennzeichnet, daß Stirn- und Wangenstützen über Längsrohre gleitverschieblich in gehäusestationären Lagerblöcken (48) aufgenommen und über Vorspannungsfedern (47) gehalten sind, mit jeweils vom zugeordneten Mikrorechner aktivierbaren Klemmvorrichtungen bei Erreichen der gewünschten Abstands- und Ausrichtposition.

27. Vorrichtung nach einem oder mehreren der Ansprüche 15-26, dadurch gekennzeichnet, daß zur Erfassung der Abstandsposition ferner einen Abstandssensor bildende, nach dem Triangulationsverfahren arbeitende Abstands-Fotodioden (60a, 60b) im Gehäusebereich vorgesehen sind, deren Ausgangssignale über den Mikrorechner die Kopfabstützungsmittel steuern.

28. Vorrichtung nach einem der Ansprüche 15-27, dadurch gekennzeichnet, daß zur Kompensation von Scherkräften zwischen der Meßfläche des Meßprismas (11) und der Augenoberfläche die in vertikaler Richtung nachgiebigen rückgeführten Federlappen (27d, 27e) der Meßfeder vorgesehen sind, die die Rohrlagerung für das Meßprisma (11) in unterschiedlicher Höhe erfassen.

29. Vorrichtung nach einem der Ansprüche 15-28, dadurch gekennzeichnet, daß ein auf eine vorgegebene rückwärtige Position des Schlittens (10) ansprechender Endpositionsschalter (52) vorgesehen ist, der den Motorstromkreis über die Mikroprozessoransteuerung unterbricht und daß dem Endpositionsschalter (52) ein mechanischer Endanschlag (53) für mindestens eines der die hintere Abschlußplatte (20') durchsetzenden Meßprisma-Tragrohre (26a) zugeordnet ist, der zur Fixierung in einer Transportposition die Tragrohrlagerung bis auf eine vordere Anschlagposition nach vorne schiebt, die von vorderen, mit den Tragrohren (26a, 26b) stationär verbundenen Anschlägen (57) bestimmt ist, die an der Innenwandung des Optikblocks (20) zur Anlage kommen.

30. Vorrichtung nach einem oder mehreren der Ansprüche 15-29, dadurch gekennzeichnet, daß als weitere Sicherheitseinrichtung ein Überlastschalter (50) vorgesehen ist, der auf eine vorgegebene rückwärts gerichtete Schwellenposition eines der Meßprisma-Tragrohre (26b) anspricht und unmittelbar den Motorstromkreis unterbricht.

31. Vorrichtung nach einem der Ansprüche 15-30, dadurch gekennzeichnet, daß die Sicherheitsvorrichtungen 1. Feststellung einer Überlast-Position durch das PSD-Element (30, 31, 31'),2. Ansprechen des Überlastschalters (50) und 3. Überwindung der Federvorspannungsreibung bei der Teleskopierbarkeit der ineinander gesteckten Tragrohre (26a, 26b, 26a', 26b') in dieser Reihenfolge ansprechen, wobei unter allen Umständen ein vorgegebener Schwellenwert (10 Pond) der auf das Auge einwirkenden Kraft nicht überschritten wird.

32. Vorrichtung nach einem der Ansprüche 15-31, dadurch gekennzeichnet, daß zur Abstandspositionierung unter Wegfall der Kopfabstützungen an den vorderen (sich verjüngenden) Endbereich des Gehäuses (12, 12a) ein vorzugsweise über ein Kardangelenk nach allen Richtungen drehbares Okular angesetzt ist, welches auf dem Augenumfeld des Gesichts aufliegt.

# Fig.1

EP 0 418 746 A1

Fig.2

## Fig.3

## Fig.4

**Fig.5**

11

**Fig.6**

11d

11'

**Fig.7**

11e

**Fig.8**

11e

# Fig.9

EP 0 418 746 A1

Fig.10

Fig.12

# Fig.11

# Fig.13

# Fig.14

EP 0 418 746 A1

Fig.15

# Fig.16

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,A | US-A-4 523 597 (S.SAWA ET AL.) <br> * das ganze Dokument * <br><br> – – – | 1,7,8,15, 2,10,16, 17 | A 61 B 3/16 |
| Y | US-A-4 621 644 (G.J.EILERS) <br> * das ganze Dokument * <br><br> – – – | 1,7,8,15 | |
| A | US-A-4 344 037 (C.W. RAGSDALE) <br> * das ganze Dokument * <br><br> – – – | 1 | |
| A | WO-A-8 803 384 (KEELER LIM.) <br> * Zusammenfassung; Figuren * <br><br> – – – | 2 | |
| A | US-A-4 192 317 (C.R. MUNNERLYN ET AL.) <br> * das ganze Dokument * <br><br> – – – | 4 | |
| A | CH-A-5 470 87 (AMERICAN OPTICAL CORPORATION) <br> * das ganze Dokument * <br><br> – – – – – | 9,17 | |
| | | | RECHERCHIERTE SACHGEBIETE (Int. Cl.5) <br><br> A 61 B |

Der vorliegende Recherchenbericht wurde für alle Patentansprüche erstellt

| Recherchenort | Abschlußdatum der Recherche | Prüfer |
|---|---|---|
| Den Haag | 12 Dezember 90 | FERRIGNO, A. |